(19) Europäisches Patentamt
European Patent Office
Office européen des brevets

(11) EP 4 474 471 A1

(12) EUROPEAN PATENT APPLICATION

(43) Date of publication:
11.12.2024 Bulletin 2024/50

(51) International Patent Classification (IPC):
C12M 1/36 (2006.01)        B01F 35/22 (2022.01)
C12M 1/00 (2006.01)        C12N 5/00 (2006.01)

(21) Application number: 23178373.9

(22) Date of filing: 09.06.2023

(52) Cooperative Patent Classification (CPC):
C12M 99/00; B01F 35/2207; C12M 41/48;
C12N 5/0018

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Sartorius Stedim Cellca GmbH
89081 Ulm (DE)

(72) Inventors:
• Safari, Ali
  89077 Ulm (DE)
• Abouzeid, Ahmed
  89134 Blaustein (DE)
• Mueller, Dirk
  70563 Stuttgart (DE)
• Huang, Yu-Chieh
  89073 Ulm (DE)
• Wieschhaus, Jörg
  89077 Ulm (DE)

(74) Representative: Cohausz & Florack
Patent- & Rechtsanwälte
Partnerschaftsgesellschaft mbB
Bleichstraße 14
40211 Düsseldorf (DE)

(54) CELL CULTURE MEDIA SPECIFICATION ATTRIBUTE ASSESSMENT

(57) Disclosed is inter alia a method comprising:
- obtaining respective impurity levels of at least one impurity in each raw material of a plurality of raw materials;
- obtaining a formulation of a cell culture medium, wherein the formulation indicates a ratio of the plurality of raw materials in the cell culture medium; and
- determining, at least partially based on the formulation and on the respective impurity levels, a total impurity level of the at least one impurity in the cell culture medium.

Fig.1

**Description**

**TECHNICAL FIELD**

Various exemplary embodiments according to the present disclosure relate to the field of cell culture, in particular to the preparation of biological growth media used for cell culture, commonly known as cell culture media. More specifically, various exemplary embodiments according to the present disclosure relate to a computer-implemented method for determining an impurity level of at least one impurity in a cell culture medium.

**BACKGROUND**

In vitro culture of cells and tissues is one of the foundations of modern biotechnology. Providing reliable and highly productive cell culture conditions allows for improvements in the production of cell culture products, for example biologicals such as therapeutic or diagnostic proteins, as well as for reliable diagnostic tests that rely on cell culture. Achieving optimal quality of a cell culture depends on the synergy between the cells to be cultured and the cell culture medium in which the cells are suspended. Accordingly, the production of cell culture media that may be tailored to a specific cell line or even to the product to be produced is of growing importance. Consequently, there is an increasing interest to precisely monitor and control the composition of cell culture media.

Cell processes often lack reproducibility, which is partly reasoned in the variability of the cell culture medium. One way to address this problem is by exchanging any less defined compound, such as animal-, yeast- or plant-derived compounds, by defined compounds, such as recombinantly produced proteins. These chemically defined media are typically characterized by significantly reduced process variability of cell culture processes carried out therewith. Nevertheless, chemically defined media still suffer from variability, as the individual compounds introduce impurities into the cell culture media, which in sum can impact the cell culture process. In addition, the impurity profile of each of these compounds again varies from supplier to supplier and from batch to batch, such that for each new sourced compound reproducibility is at stake. While some approaches in the art aim at reducing the impurities co-introduced into the cell culture media with the chemical compounds, the purification and production processes of such purer compounds renders these alternative approaches more laborious and thus more expensive.

**SUMMARY OF SOME EXEMPLARY EMBODIMENTS**

According to the present disclosure, a computer-implemented method is disclosed, wherein the method comprises:

- obtaining respective impurity levels of at least one impurity in each raw material of a plurality of raw materials;
- obtaining a formulation of a cell culture medium, wherein the formulation indicates a ratio of the plurality of raw materials in the cell culture medium; and
- determining, at least partially based on the formulation and on the respective impurity levels, a total impurity level of the at least one impurity in the cell culture medium.

Further according to the present disclosure, a computer program is disclosed, wherein the computer program comprises instructions which, when the computer program is executed by a computer, cause the computer to carry out the method according to the present disclosure. For example, the computer program comprises program instructions which cause a processor to perform and/or control the method according to the present disclosure when the computer program runs on the processor. For example, a processor is intended to be understood as meaning, inter alia, control units, microprocessors, microcontrol units such as microcontrollers, digital signal processors (DSP), application-specific integrated circuits (ASICs) or field programmable gate arrays (FPGAs). In this case, either all steps of the method can be controlled or all steps of the method can be performed or one or more steps can be controlled and one or more steps can be performed. The computer program may be distributable, for example, via a network such as the Internet, a telephone or mobile radio network and/or a local area network. The computer program may be at least partially software and/or firmware of a processor. It may likewise be at least partially implemented as hardware. The computer program may be stored, for example, on a computer-readable storage medium, for example a magnetic, electrical, optical and/or other type of storage medium. The storage medium may be, for example, part of the processor, for example a (nonvolatile or volatile) program memory of the processor or a part thereof. The storage medium may be a tangible or physical storage medium, for example.

Further according to the present disclosure, an apparatus is disclosed, wherein the apparatus is configured to perform and/or control the method according to the present disclosure. Alternatively, the apparatus may comprise respective means (e.g. computer means) for performing and/or controlling the steps of the method according the present disclosure. In this case, either all steps of the method can be controlled or all steps of the method can be performed or one or more steps can be controlled and one or more steps can be performed. One or more of the steps may also be performed and/or controlled by the same unit For example, one or more of the steps may be performed by one or more processors.

Further according to the present disclosure, an apparatus comprising at least one processor and at least one memory including program code is disclosed, wherein the at least one memory and the program code are configured to cause the apparatus with the at least one processor to perform and/or control at least the method according to the present disclosure. In this case, either all steps of the method can be controlled or all steps of the method can be performed or one or more steps can be controlled and one or more steps can be performed.

For example, a cell culture medium is meant to be understood as a solid or liquid composition comprising or consisting of a plurality of raw materials. In further examples, a cell culture medium may contain a solvent such as water in addition to the plurality of raw materials. For example, a raw material is meant to be understood as an ingredient of a cell culture medium, which may for example be a chemical compound, in particular an organic or inorganic compound, an inorganic salt, an organic salt, a singular amino acid or mixture of multiple different amino acids, a peptide, a protein, a sugar or mixture of multiple sugars, a sugar alcohol, an oligo- or polysaccharide or mixture of multiple oligo- and/or polysaccharides, a nucleic acid or mixture of multiple nucleic acids, an amino acid derivative, a biogenic amine, a vitamin, a fatty acid, a buffering agent, a dye or a biological substance such as a serum such as for example a fetal bovine serum (FBS). For example, a raw material may be present as a solid (e.g. a powder) or as a liquid. A raw material may for example be fluorescently or radioactively labeled. As further described below, a raw material may contain one or more impurities.

For example, an impurity is meant to be understood as a substance that is present in a raw material and may not be the designated compound of said raw material. Exemplary types or categories of impurities in respective raw materials of cell culture media are elemental impurities, inorganic compounds, organic compounds, endotoxin or bioburden. Elemental impurities may in particular refer to respective chemical elements. In further examples, impurities, in particular, but not limited to, elemental impurities, may be toxic impurities and/or functional impurities. Impurities may for example be solvents or compounds derived from solvents. For example, an impurity may also be referred to as trait, contaminant, defect or similar.

For example, in the context of impurities, the terms "chemical element" and "element" are meant to be understood as a chemical substance defined by its atomic number. Within the present disclosure, the elemental symbol as present in the periodic table of elements may be used to refer to an element in any charged, uncharged, or complexed forms. For example, an elemental impurity may be present in the form of an uncharged elemental particle, a salt, an ion, a complexed compound, a coordination compound, or the like.

For example, a toxic impurity is meant to be understood as an elemental impurity that, when being present in a particular amount or respective concentration (e.g. an amount or a concentration above a particular limit) in a cell culture medium, may cause adverse effects such as lower yield of cell culture product, reduced cell growth, cell death, contamination of a cell culture product, alteration of product quality attributes or the like. For example, it may be preferred that the amount of a toxic impurity in a cell culture medium shall be below an upper limit. Without being limited thereto, a toxic impurity may, for example, be Pb, Hg, As, Cd, Ag, Cr, Li, Sb, or Ba or combinations thereof. The term "a" as used herein (as in "a toxic impurity") shall be understood to mean "one or more" unless the context clearly provides otherwise.

For example, a functional impurity is meant to be understood as an elemental impurity that, when being present in a particular amount or respective concentration (e.g. an amount or a concentration above a lower limit and/or below an upper limit) in a cell culture medium, may provide a benefit to a cell culture and/or may even be at least partially essential for a functioning cell culture. In further examples, a functional impurity may, for example, when being present in a particular amount or a respective concentration above an upper limit or below a lower limit, cause adverse effects such as those stated above for toxic impurities.

Therefore, it may for example be preferred that functional impurities are present in a cell culture medium in an amount or concentration which may be above a lower limit and below an upper limit Without being limited thereto, a functional impurity may for example be V, Se, Ca, K, Mg, P, S, Sn, Na, Co, Fe, Cu, Mn, Mo, Ni, or Zn or combinations thereof.

For example, in the context of impurities, an organic compound is meant to be understood as an organic chemical compound which, when present in a particular amount or respective concentration (e.g. an amount or a concentration above a particular limit) in a cell culture medium, may cause adverse effects such as lower yield of cell culture product, reduced cell growth, cell death, contamination of a cell culture product, alteration of product quality attributes or the like. For example, it may be preferred that the amount of an organic compound in a cell culture medium shall be below an upper limit An organic compound may for example be dimethyl sulfoxide (DMSO), acetonitrile, methanol, ethanol, formaldehyde, glycerol, polyethylene glycol or the like.

For example, in the context of impurities, an inorganic compound is meant to be understood as an inorganic chemical compound which, when present in a particular amount or respective concentration (e.g. an amount or a concentration above a particular limit) in a cell culture medium, may cause adverse effects such as lower yield of cell culture product, reduced cell growth, cell death, contamination of a cell culture product, alteration of product quality attributes or the like. For example, it may be preferred that the amount of an inorganic compound in a cell culture medium shall be below an upper limit An inorganic compound may for example be an inorganic salt such as copper sulfate, zinc chloride or silver nitrate, arsenic chloride, antimony chloride or an inorganic acid or base such as hydrochloric acid, sulfuric acid, sodium hydroxide, potassium hydroxide or the like.

For example, endotoxin is meant to be understood as a chemical or biological by-product of raw material production, if for example said production takes place in a biological organism. Because endotoxins may often provide no benefit to a cell culture or even be harmful, it may be preferred that an amount or concentration of endotoxin in a cell culture medium does not exceed an upper limit Without being limited thereto, an endotoxin may for example be at least one lipopolysaccharide, or at least one bacterial toxin.

For example, bioburden is meant to be understood as any archaeic, prokaryotic, eukaryotic or viral biological unit present within a raw material or respective cell culture medium. Because bioburdens may provide no benefit to a cell culture or even be harmful, it may for example be preferred that the amount of a bioburden in a cell culture medium does not exceed an upper limit. Without being limited thereto, a bioburden may for example be a mycoplasm, coccus, yeast, fungus or bacillus.

For example, an impurity level is meant to be understood as an amount or concentration of a corresponding impurity within a raw material and/or cell culture medium. For example, the impurity level of a certain impurity in a cell culture medium may be determinable at least partially based on an impurity level of said impurity in one or more raw materials included in the cell culture medium.

Obtaining respective impurity levels or a formulation of a cell culture medium may for example be understood to mean that information, parameter and/or data representing the respective impurity levels or the formulation (e.g. in a suitable electronic format such as a text format, spreadsheet format or database format) may be received at an apparatus performing the method (e.g. by means of a communication interface of the apparatus e.g. from another apparatus providing a raw material database or a formulation database). For example, such receiving may be caused or instructed by requesting the respective impurity levels or the formulation of a cell culture medium. In another example, obtaining respective impurity levels may comprise causing the measuring of at least one impurity level, for example by requesting another apparatus to measure and subsequently transmit the particular impurity level.

Determining a total impurity level of the at least one impurity in the cell culture medium at least partially based on the formulation and on the respective impurity levels may for example be understood to mean that the total impurity level may be calculated or computed, wherein such calculating or computing may at least partially depend on the formulation and on the respective impurity levels.

A plurality of raw materials may for example be understood as raw materials that are used to prepare a cell culture medium and that are included in the cell culture medium after the cell culture medium has been prepared. For example, a cell culture medium may comprise at least 20 to 30 or up to 150 raw materials, although cell culture media having any number of raw materials (e.g. at least 2, at least 3, at least 4, at least 5, at least 10, at least 20, or at least 30 raw materials) may be subject of the method of the present disclosure. The ratios according to which the plurality of raw materials is included in the cell culture medium may be indicated by a formulation of the cell culture medium. For example, the plurality of raw materials may correspond to all raw materials or to a subset of all raw materials included in the cell culture medium.

For example, a formulation of a cell culture medium (e.g. a recipe of a cell culture medium) is meant to be understood as a specific weight ratio, molar ratio, or concentration measure based on mass or molar amount per volume of a plurality of raw materials contained in the cell culture medium. In some examples, a formulation may indicate the ratio of the plurality of raw materials in a relative manner (e.g. by indicating that the content of raw material A is 20 % and the content of raw material B is 80 % in the cell culture medium) or in an absolute manner (e.g. by indicating the absolute content of raw material A and raw material B in the cell culture medium). Therein, for example, it may be preferred that a percentage corresponds to a weight-percentage, for example such that a raw material content in a cell culture medium of 20 % corresponds to 20 wt-%, wherein 100 % or 100 wt.-% may correspond to the dry weight of the cull culture medium or respectively to the weight of the cell culture medium prior to hydration. For example, a formulation of a cell culture medium may refer to a particular amount to the cell culture medium. A formulation may for example be obtained by obtaining (e.g. receiving or determining) information, parameter and/or data representing the formulation (e.g. in a suitable electronic format such as a text format, spreadsheet format or database format).

An impurity level of an impurity in a raw material may for example be understood as the amount of this particular impurity in the raw material (e.g. in a particular amount of the raw material). For example, a raw material may include a plurality of impurities, whose respective amounts in the raw materials are given by respective impurity levels. An impurity level may for example be obtained by obtaining (e.g. receiving or determining) information, parameter and/or data representing the impurity level (e.g. in a suitable electronic format such as a text format, spreadsheet format or database format).

An impurity level may be expressed in various manners. In one example, an impurity level may be expressed as parts per million (ppm) representing the number of parts of the impurity per million parts of the raw material. In another example, an impurity level may be expressed as weight-percent representing the weight of the impurity as a percentage of the total weight of the raw material. In another example, an impurity level may be expressed as mole fraction representing the ratio of the number of moles of the impurity and the number of moles of the raw material.

A total impurity level of an impurity in the cell culture medium may for example be understood as the amount of this particular impurity in the cell culture medium (e.g. in a particular amount of the cell culture medium). For example, the cell culture medium may include a plurality of impurities, whose respective amounts in the cell culture medium are given by respective total impurity levels. After determining the total impurity level, the total impurity level may for example be indicated by corresponding information, parameter and/or data (e.g. in a suitable electronic format such as a text format, spreadsheet format or database format).

The total impurity level of at least one impurity in the cell culture medium may be affected by the impurity level of the at least one impurity in each raw material of the plurality of raw materials in the cell culture medium. In other words, depending on the respective impurity levels of the at least one impurity in each raw material and the ratio of the plurality of raw materials in the cell culture medium as indicated by the formulation, the total impurity level may be determined. The total impurity level may then be understood as total amount of the impurity in the cell culture medium as given by the sum of the respective amounts of the impurity in each raw material.

Advantageously, by determining (e.g. calculating or computing) a total impurity level of at least one impurity in the cell culture medium, the selection of one or more raw materials included in the cell culture medium may be optimized. For example, depending on whether the determined total impurity level meets a predefined criterion, it may be determined whether optimization of the raw material selection is needed (e.g. by replacing one or more raw materials of a particular batch by another batch of the respective raw material, which other batch has a different respective impurity level of the at least one impurity, or e.g. by replacing one or more raw materials by respective equivalent raw materials that have a different counter ion and/or different level of hydration) to change the total impurity level in the cell culture medium. Since determining the total impurity level depends on the formulation and the respective impurity levels of each raw material of the plurality of raw materials, the contribution of each raw material according to the raw materials content in the cell culture medium may be taken into account for assessing the raw material selection. For example, in an embodiment encompassing replacing one or more raw materials by respective equivalent raw materials that have a different counter ion, it may be preferred that the counter ion does not significantly change the respective element content in the composition or, preferably, is balanced by a corresponding change in another raw material to result in the same ion content in the cell culture medium.

In the following, further exemplary features and exemplary embodiments according to the present disclosure will be described in more detail.

According to an exemplary embodiment, the method of the present disclosure further comprises:

- determining, at least partially based on the formulation and on respective impurity levels of at least one further impurity in each raw material of the plurality of raw materials, at least one further total impurity level of the at least one further impurity in the cell culture medium.

For example, the at least one further impurity may be, independently from the at least one impurity, selected from the group consisting of elemental impurities, inorganic compounds, organic compounds, endotoxin and bioburden.

For example, at least one further impurity is meant to be understood as encompassing any number of further impurities, for example at least two further impurities, at least three further impurities, at least 4, at least 5, at least 6, at least 7, at least 8, at least 9, at least 10, at least 15, or at least 20 further impurities. Therein, for example, each of these further impurities may be independently selected, such that, for example, the further impurities may encompass at least one elemental impurity and/or at least one inorganic compound and/or at least one organic compound and/or at least one endotoxin and/or at least one bioburden.

For determining the at least one further total impurity level of at least one further impurity, the respective impurity levels of this at least one further impurity in each raw material of the plurality of raw materials are considered. The method according to the present disclosure may for example comprise obtaining the respective impurity levels of the at least one further impurity in each raw material of the plurality of raw materials.

By determining at least one further total impurity level of at least one further impurity in addition to the at least one impurity, a plurality of impurities may be taken into account for assessing the raw material selection. This may for example cover scenarios where in one raw material of the plurality of raw materials, the impurity level of the at least one impurity is rather high, while the impurity level of the at least one further impurity is rather low. In examples where different criteria (e.g. by different upper and/or lower limits) may apply for the total impurity level of the at least one impurity and the total impurity level of the at least one further impurity, the method of the present disclosure may be used to identify a raw material selection complying with all of these different criteria.

For example, the method of the present disclosure has the advantage that impurity levels of a plurality of impurities such as, for example, at least one toxic impurity and at least one further toxic impurity and/or at least one functional impurity and/or at least one endotoxin and/or at least one bioburden and/or at least one inorganic compound and/or at least one organic compound, may be determined in a single optimization step.

According to an exemplary embodiment, the method of the present disclosure further comprises:

- causing measuring at least one impurity level of the respective impurity levels in each raw material of the plurality of raw materials; and/or
- outputting information indicating the total impurity level of the at least one impurity in the cell culture medium and/or the at least one further total impurity level of the at least one further impurity in the cell culture medium.

For example, causing measuring at least one impurity level of the respective impurity levels in each raw material of the plurality of raw materials may be understood to mean that the apparatus performing the method may cause a measurement of the at least one impurity level by sending an electrical signal for instructing a measurement apparatus to measure and subsequently transmit the respective impurity levels.

Outputting information indicating the total impurity level may for example be understood to mean that an apparatus performing the method may provide information, parameter and/or data representing the total impurity level and/or the at least one further total impurity level (e.g. in a suitable electronic format such as a text format, spreadsheet format or database format) for example by means of a communication interface of the apparatus. In a non-limiting example, a user interface may be used for outputting respective total impurity levels to a user (e.g. by presenting the total impurity levels on a screen as visual user output).

According to an exemplary embodiment of the method of the present disclosure, the respective impurity levels in each raw material refer to a respective batch of the respective raw material.

For example, a batch of raw material is meant to be understood as a specific quantity or lot of the raw material that may have been produced or manufactured under the same set of conditions or procedures, including the specific formulation, processing steps, equipment, and environmental conditions required to manufacture the raw material. For example, within a single batch of a raw material, an impurity level of one or more impurity in the raw material is considered to be constant When comparing for example an impurity level of a particular impurity in a raw material for several batches of this raw material, the impurity level of this particular impurity may vary among the several batches of this raw material. For example, different batches of a respective raw material may originate from different suppliers or from the same supplier.

According to an exemplary embodiment, the method of the present disclosure further comprises:

- determining, at least partially based on the total impurity level in the cell culture medium, the formulation and the respective impurity levels, a respective contribution of one or more raw materials of the plurality of raw materials to the total impurity level and/or to the at least one further total impurity level in the cell culture medium.

For example, a contribution of a raw material to the total impurity level of an impurity may be a ratio of (i) the impurity level of the impurity in the raw material and (ii) the total impurity level of this impurity in the cell culture medium by further including the proportion of the raw material in the cell culture medium. For example, the contribution may be expressed as percentage, wherein with respect to the same impurity, the respective contributions of all raw materials of a cell culture medium may add up to 100 percent

For example, a first raw material may have a larger contribution to a total impurity level compared to a second raw material (e.g. because of a higher impurity level of the impurity in the first raw material than in the second raw material and/or because a higher amount of the first raw material is included in the cell culture medium than of the second raw material). In such an example, the first raw material may be referred to as sensitive raw material.

Considering for example respective total impurity levels of a plurality of impurities in a cell culture medium including a plurality of raw materials, a matrix representation (e.g. a sensitivity matrix) may provide the contribution of each raw material to each of the total impurity levels.

Advantageously, the method of the present disclosure may allow for rank-ordering of raw materials with respect to their contribution to total impurity levels, which in turn improves the capability of the method to efficiently determine those raw materials which primarily contribute to a certain total impurity level in the cell culture medium.

According to an exemplary embodiment, the method of the present disclosure further comprises:

- outputting information indicating one or more raw materials whose contribution to the total impurity level and/or to the at least one further total impurity level is larger than a predefined threshold.

Outputting information indicating the total impurity level may for example be understood to mean that an apparatus performing the method may provide information, parameter and/or data representing the one or more raw materials (e.g. in a suitable electronic format such as a text format, spreadsheet format or database format) for example by means of a communication interface of the apparatus. In a non-limiting example, a user interface may be used for outputting the one or more raw materials to a user (e.g. by presenting the one or more raw materials on a screen as visual user output).

For example, information indicating one or more raw materials whose contribution to the total impurity level and/or to the at least one further total impurity level is larger than a predefined threshold may be used to represent a short list of sensitive raw materials out of the plurality of raw materials that have a particularly strong influence on the total impurity level in the cell culture medium. Advantageously, only these sensitive raw materials may be used when further assessing (e.g. optimizing) a total impurity level in the cell culture medium. For example, any further analysis such as determining an allowable range or determining a respective preferred batch may only be based on these sensitive raw materials, which may make the analysis (e.g. a corresponding optimization algorithm) more efficient

According to an exemplary embodiment, the method of the present disclosure further comprises:

- determining whether the total impurity level and/or the at least one further total impurity level meet at least one predefined criterion.

For example, a total impurity level of a particular impurity in the cell culture medium may meet a predefined criterion of the cell culture medium by being below or above a limit for the total impurity level of this impurity in the cell culture medium. Considering an example of a plurality of total impurity levels in the cell culture medium, respective different predefined criteria may apply for each of the total impurity levels. A criterion may for example be predefined according to regulations existing with respect to the physical, chemical, biological, or microbiological characteristics of the cell culture medium, wherein these regulations and corresponding criteria need to be met to ensure the safety, efficacy, and quality of the cell culture medium or any product prepared at least partially based on the cell culture medium.

According to an exemplary embodiment of the method of the present disclosure, determining whether the total impurity level and/or the at least one further total impurity meet at least one predefined criterion further comprises at least one of the following:

- determining whether the total impurity level and/or the at least one further total impurity level are below a predefined upper limit in the cell culture medium; and/or
- determining whether the total impurity level and/or the at least one further impurity total level are above a predefined lower limit in the cell culture medium.

For example, an upper limit is meant to be understood as a predetermined maximum amount or maximum concentration of an impurity in the cell culture medium, above which detrimental effects on the cell culture and/or cell culture product may be expected. For example, a total impurity level of any impurity above a respective upper limit of this impurity in the cell culture medium should be avoided. For example, the upper limit may be based on the impurity and/or the cell culture medium. For example, each impurity in a cell culture medium may have an individual upper limit Further, the upper limit of an impurity may differ or be the same with respect to different cell culture media.

For example, a lower limit is meant to be understood as a predetermined minimum amount or minimum concentration of an impurity in the cell culture medium, below which detrimental effects on the cell culture and/or cell culture product can be expected. Therein generally, a lower limit is appreciated for a functional impurity, wherein a total impurity level of any functional impurity below a respective lower limit in the cell culture medium should be avoided. For example, the lower limit may be based on the impurity and/or the cell culture medium. For example, each impurity in a cell culture medium may have an individual lower limit. Further, the lower limit of an impurity may differ or be the same with respect to different cell culture media. Advantageously, the method of the present disclosure may allow for individually tailored limits with respect to different cell culture media and/or different impurities. This may for example cover scenarios where the total impurity level of an undesirable impurity (e.g. a toxic impurity) shall be below an upper limit, while for another the total impurity level of a desirable impurity (e.g. a functional impurity) shall be above a lower limit

According to an exemplary embodiment of the method of the present disclosure, determining whether the total impurity level and/or the at least one further total impurity level meet at least one predefined criterion is at least partially based on an upstream process model and/or on a downstream process model of the cell culture medium.

For example, an upstream process model is meant to be understood as a model or calculation of the impurity level in the cell culture medium and/or cell culture product on the basis of an upstream process. Therein, for example, an upstream process is meant to be understood as the process leading up to and including the production of a cell culture product by cells in a cell culture medium. For example, the upstream process may comprise activities such as cell culture, and/or a process including time-variant addition of different cell culture media (e.g. feed media), and/or partial removal of cell culture medium (e.g. by at least one sampling step) and/or addition of further substances during a cell culture process (e.g. for the purpose of pH regulation, foam prevention or the like).

For example, a downstream process model is meant to be understood as a model or calculation of the impurity level in the cell culture product on the basis of a downstream process. Therein, for example, a downstream process is meant to be understood as the process of purifying the cell culture product and preparing it for later use. For example, the downstream process may comprise activities such as cell harvesting, cell disruption, chromatography, filtration and/or formulation (e.g. by including such activities as a series of unit operations).

Advantageously, by considering an upstream and/or downstream process model, the method of the present disclosure may allow for a more precise estimation of the total impurity levels of the at least one impurity in the cell culture medium and/or cell culture product produced therewith, which in turn allows for a more accurate prediction of whether total impurity levels may meet respective predefined criteria and thus whether the cell culture product will comply with regulations such as critical quality attributes (CQA). The additional consideration of the upstream and/or downstream process may in some examples provide more lenient limits for the total impurity levels in the cell culture medium and thus may affect the raw material selection.

According to an exemplary embodiment, the method of the present disclosure further comprises:

- if it is determined that the total impurity level and/or the one further total impurity level meet the at least one predefined criterion, outputting information indicating the plurality of raw materials and the formulation as suitable for manufacturing the cell culture medium.

Outputting information indicating the plurality of raw materials and the formulation as suitable for manufacturing the cell culture medium may for example be understood to mean that an apparatus performing the method may provide information, parameter and/or data representing that the plurality of raw materials are suitable used for manufacturing a cell culture medium (e.g. in a suitable electronic format such as a text format, spreadsheet format or database format) for example by means of a communication interface of the apparatus. In a non-limiting example, a user interface may be used for outputting the one or more raw materials to a user (e.g. by presenting the one or more raw materials on a screen as visual user output).

In one further example, if it is determined that the total impurity level and/or the one further total impurity level meet the at least one predefined criterion, the method may further comprise causing manufacturing (e.g. by instructing or controlling a manufacturing apparatus) a cell culture medium using the plurality of raw materials and the formulation indicated as suitable.

According to an exemplary embodiment, the method of the present disclosure further comprises:

- determining, at least partially based on the formulation, on the respective impurity levels in each raw material of the plurality of raw materials and on at least one predefined criterion, a respective allowable range of the impurity level of the at least one impurity in at least one raw material of the plurality of raw materials.

For example, it may be determined that the impurity level of at least one impurity in at least one raw material may vary within an allowable range, while the total impurity level of this first impurity in the cell culture medium still meets a predefined criterion (e.g. an upper and/or lower limit). In other words, a total impurity level of the at least one impurity in the cell culture medium, determined at least partially based on the formulation and any impurity level of the at least one impurity in the at least one raw material within the determined allowable range, meets the at least one predefined criterion. For example, under variation within the allowable range of an impurity level in a raw material, the at least one predefined criterion is still met with a particular probability. Therein, an allowable range for an impurity level of a particular impurity may be determined to ensure that the probability of failing a predefined criterion for a total impurity level of this impurity may be below a threshold probability.

In another example, determining an allowable range of an impurity level of a particular impurity may not be based on the respective impurity levels of this particular impurity in each raw material of the plurality of raw materials in the cell culture medium, but rather on the respective impurity levels of this particular impurity in those raw materials for which it has been determined that their contribution to a total impurity level of this particular impurity is larger than a predefined threshold. This way, the efficiency of an optimization algorithm that may for example be used for determining the allowable range may be improved.

An allowable range of an impurity level in a raw material may for example be given by an upper and lower limit for this impurity level, wherein this upper and lower limit may be understood as allowable boundaries for this impurity level.

Advantageously, the method according to the present disclosure provides allowable ranges on impurity levels for particular raw materials, wherein it is ensured that predefined criterions for the cell culture medium are met (e.g. with a particular probability) as long as the impurity levels in the raw materials vary within the allowable range. Considering for example that impurity levels in raw materials may vary for different batches of this raw material, allowable ranges advantageously indicate whether a different batch of a raw material may still be used for manufacturing a cell culture medium or whether it should be discarded.

According to an exemplary embodiment, determining the respective allowable range of the impurity level of the at least one impurity in at least one raw material of the plurality of raw materials further comprises:

- calculating, at least partially based on an initial range of the impurity level of the at least one impurity in the at least one raw material, a probability that the total impurity level of the at least one impurity in the cell culture medium fails the at least one predefined criterion; and
- determining, in particular by using an optimization algorithm, the respective allowable range of the impurity level based on the calculated probability and the initial range of the impurity level.

For example, an initial range of an impurity level of a particular impurity level in a raw material may be determined based on statistical variations for this impurity level among various batches of this raw material. An initial range may for example be retrieved from a raw material data base.

Calculating a probability that the total impurity level of the at least one impurity in the cell culture medium fails or violates the at least one predefined criterion may for example comprise selecting a plurality of impurity levels (e.g. as samples) of the at least one impurity within the initial range of the impurity level of this impurity in at least one raw material. Subsequently, it may be determined for how many of these selected impurity levels within the initial range the at least one predefined criterion may be violated. For example, the ratio between the impurity levels for which the predefined criterion is violated compared to the plurality of all selected impurity levels within the allowable range may indicate the probability of violating the at least one predefined criterion.

Subsequently, the probability of violating the at least one predefined criterion may be compared to a threshold probability, which for example indicates whether a certain violation probability is still acceptable or not. If the violation probability is above the threshold probability, the initial range may be decreased to arrive at the allowable range. If the violation probability is below the threshold probability, the initial range may be increased to arrive at the allowable range.

In further examples, determining the allowable range based on the initial range may comprise performing an optimization algorithm, which aims at minimizing an objective function including the difference between the violation probability and the threshold probability. Such an optimization algorithm may for example be performed for several iterations, wherein an allowable range as output of one iteration may be used as input (e.g. as initial range) for the subsequent iteration of the optimization algorithm.

For example, an initial range of the impurity level of the at least one impurity may be given by a mean value and a standard deviation for this mean value. Then, determining the respective allowable range of the impurity level based on the calculated probability and the initial range of the impurity level may comprise determining a mean value and/or a standard deviation of the initial range.

According to an exemplary embodiment, the method of the present disclosure further comprises:

- outputting information indicating the respective allowable range of the impurity level in the at least one raw material.

Outputting information indicating the respective allowable range may for example be understood to mean that an apparatus performing the method may provide information, parameter and/or data representing the allowable range (e.g. in a suitable electronic format such as a text format, spreadsheet format or database format) for example by means of a communication interface of the apparatus. In a non-limiting example, a user interface may be used for outputting the one or more raw materials to a user (e.g. by presenting the one or more raw materials on a screen as visual user output).

According to an exemplary embodiment, the method of the present disclosure further comprises:

- determining a deviation of the total impurity level and/or the at least one further total impurity level in the cell culture medium from a respective reference total impurity level in the cell culture medium.

For example, a deviation of a total impurity level of a particular impurity is meant to be understood as the amount or extent by which the total impurity level differs or deviates from a reference total impurity level of this particular impurity in the cell culture medium. Therein, for example, the reference total impurity level may refer to an allowable range and/or upper limit and/or lower limit of the corresponding impurity in the cell culture medium. For example, it may be preferred that if the total impurity level and/or the at least one further total impurity level in the cell culture medium is determined to be within the allowable range and/or below the upper limit, optionally above the lower limit, the deviation is determined to be zero. Advantageously, the method according to the present disclosure may allow for accurately tailoring the requirements of the cell culture process to be optimized.

For example, an objective function may be used to express a deviation of the total impurity level from a respective reference total impurity level. Such an objective function may additionally express a deviation of at least one further total impurity level from a respective at least one further reference total impurity level. In this example, minimizing the objective function may advantageously take into account potential deviations of a plurality of total impurity levels from respective reference total impurity levels.

According to an exemplary embodiment, the method of the present disclosure further comprises:

- determining, at least partially based on the total impurity level and/or the at least one further total impurity in the cell culture medium, the formulation and the respective impurity levels in each raw material, a respective contribution of one or more raw materials of the plurality of raw materials to the determined deviation; and

- determining one or more raw materials whose contribution to the determined deviation is larger than a predefined threshold.

For example, a contribution of a raw material to the determined deviation of the total impurity level from a respective reference total impurity level in the cell culture medium may be given by the contribution of this raw material to the total impurity level for which the deviation is determined. Therein the contribution to the total impurity level may for example be determined as further described above.

Advantageously, the method of the present disclosure may allow for rank-ordering of raw materials with respect to their contribution to a deviation of a total impurity level from a respective reference total impurity level, which may improve the capability of the method to efficiently determine those raw materials which need to be optimized to ensure that for example a total impurity level may meet a predefined criterion.

For example, by determining one or more raw materials whose contribution to the determined deviation is larger than a predefined threshold, a culprit list of sensitive raw materials out of the plurality of raw materials that have a particular strong influence on a deviation of a total impurity level in the cell culture medium may be identified.

According to an exemplary embodiment, the method of the present disclosure further comprises:

- determining a respective preferred batch of at least one raw material of the plurality of raw materials, wherein, at least partially based on the impurity level of the at least one impurity in the preferred batch of the at least one raw material, a deviation of the total impurity level and/or the at least one further total impurity level in the cell culture medium from a respective reference total impurity level in the cell culture medium is reduced, in particular by using an optimization algorithm for minimizing the deviation of the total impurity level and/or the at least one further total impurity level from the respective reference total impurity level.

Considering for example the plurality of raw materials included in the cell culture medium, each raw material may refer to a respective batch. In such an example, reducing a deviation of a total impurity level from a respective reference total impurity level in the cell culture medium by determining a respective preferred batch of at least one raw material may be understood to mean that for the at least one raw material, an alternative batch is determined based on which the deviation of the total impurity level of a particular impurity is reduced (e.g. minimized). For example, the impurity level of this particular impurity in the alternative raw material batch may be different from (e.g. higher or lower) than the impurity level of this particular impurity in an original raw material batch, based on which the deviation of the total impurity level of the particular impurity has been determined. In this case, the alternative batch may be referred to as the preferred batch. Such a difference in impurity level between the original batch and the preferred batch may for example cause the total impurity level in the cell culture medium to change in a way that leads to reducing the deviation of the total impurity level.

According to an exemplary embodiment, a raw material which is equivalent (e.g. referred to as equivalent raw material) to the at least one raw material may be determined (e.g. instead of a preferred batch of at least one raw material), wherein, at least partially based on the impurity level of the at least one impurity in the equivalent raw material, a deviation of the total impurity level and/or the at least one further total impurity level in the cell culture medium from a respective reference total impurity level in the cell culture medium is reduced.

In such an example, the method of the present disclosure further comprises:

- determining a respective equivalent raw material of at least one raw material of the plurality of raw materials, wherein, at least partially based on the impurity level of the at least one impurity in the respective equivalent raw material, a deviation of the total impurity level and/or the at least one further total impurity level in the cell culture medium from a respective reference total impurity level in the cell culture medium is reduced, in particular by using an optimization algorithm for minimizing the deviation of the total impurity level and/or the at least one further total impurity level from the respective reference total impurity level.

For example, such an equivalent raw material of the at least one raw material may be chemically and biologically equivalent to the at least one raw material, which may be understood to mean that substituting the at least one raw material by the equivalent raw material in the cell culture medium would have no substantial side effects. For example, in an equivalent raw material, the level of hydration may be different (e.g. by having a higher or lower solvent content) or a counter ion may be exchanged, for example if the exchange of the counter ion has no adverse effect on the cell culture and/or the cell culture product, wherein, for example, the counter ion does not significantly change the respective element content in the composition or, preferably, the exchange is accompanied by a corresponding change in another raw material to result in essentially the same ion content in the cell culture medium as compared to the cell culture medium without substituting the raw material. This may have the advantage that a larger choice of essentially the same raw material is available, as well as a larger choice of suitable impurity profiles.

For example, minimizing the deviation from the respective reference total impurity level may be understood to mean that the deviation is minimized to be within a range around the total reference impurity level.

For example, an objective function may be used to express the deviation between a determined total impurity level and a total reference impurity level. Reducing the deviation may then for example be achieved by identifying a minimum of the objective function. In one example, a preferred batch of a raw material may be determined by calculating the objective function for every possible combination of batches of the plurality of raw materials. The preferred batch of the particular raw material may then be determined from the combination of batches that leads to the minimum value of the objective function. To improve the efficiency of such an approach if a larger number of raw materials (e.g. 50 raw materials) in a cell culture medium with respective batches is assessed, not each raw material of the plurality of raw materials may be considered, but only those raw materials whose contribution to the corresponding total impurity level is larger than a predefined threshold. Another approach of improving efficiency when determining a preferred batch may be to use a global optimizer for minimizing the objective function and determining the best combination of batches. Such an approach may for example involve a conversion function used for solving a discrete problem (e.g. the selection of a preferred batch), while minimizing the objective function may require solving a continuous optimization problem. Advantageously, the method of the present disclosure may allow for selecting batches of raw materials which allow the resulting cell culture medium and cell culture product to comply with standards such as ICH guidelines and/or with CQAs for a given cell line, cell culture medium and/or process.

According to an exemplary embodiment of the method of the present disclosure, a respective preferred batch of the one or more raw materials whose contribution to the determined deviation is larger than a predefined threshold is determined.

Considering an example where preferred batches for reducing a deviation of a total impurity level are determined for several raw materials of the plurality of raw materials, respective preferred batches may only be determined for those raw materials as sensitive raw materials for which it has been determined that their contribution to the determined deviation is larger than a predefined threshold. Advantageously, it may already suffice to replace original batches for those sensitive raw materials by respective preferred batches for reducing the deviation such that a predefined criterion is met In such an example, the efficiency of an optimization algorithm determining the preferred batches may be improved, because it may skip those raw materials that have a rather low contribution to the deviation to be reduced.

According to an exemplary embodiment of the method of the present disclosure, a plurality of formulations of respective cell culture media is obtained, wherein each formulation of the plurality of formulations indicates a ratio of a plurality of raw materials in the respective cell culture medium; and wherein the method further comprises at least one of the following:

- determining the respective allowable range of the impurity level of the at least one impurity based on the plurality of formulations; and/or
- determining a respective preferred batch of the at least one raw material based on the plurality of formulations.

For example, the plurality of cell culture media may be understood as portfolio of cell culture media, which may be prepared based on a respective plurality of formulations, which may indicate a ratio of a plurality of raw materials in the respective cell culture medium. While these formulations as well as the pluralities of raw materials may differ from each other for the respective cell culture media of the portfolio, the respective cell culture media may for example at least share one or more common raw materials. In such an example, a respective allowable range of the impurity level in such common raw materials and/or preferred batches of such common raw materials may be determined. In such a case, determining the respective allowable range and/or determining a respective preferred batch may rely on the respective formulations and further on the respective plurality of raw materials of the cell culture media in the portfolio. Advantageously, when optimizing a whole portfolio of cell culture media, the method according to the present disclosure may for example ensure that predefined criteria are met for all media in the portfolio. This approach may ensure that as few raw materials as possible are endowed with specification limits that pose additional effort in the sourcing process for production.

According to an exemplary embodiment of the method of the present disclosure, the at least one impurity and/or the at least one further impurity is one of the following:

- a toxic impurity; or
- a functional impurity; or
- an organic compound; or
- an inorganic compound; or
- bioburden; or
- endotoxin.

Considering for example one single impurity, this impurity may be a toxic impurity, or a functional impurity, or an organic compound, or an inorganic compound, or bioburden or endotoxin. In another example considering one impurity (i.e. as first impurity) and one further impurity (i.e. as second impurity), the first impurity may be a toxic impurity, or a functional impurity, or an organic compound, or an inorganic compound, or bioburden or endotoxin, and the second impurity may be a toxic impurity, or a functional impurity, or an organic compound, or an inorganic compound, or bioburden or endotoxin. In yet another example considering one further impurity (i.e. as third impurity), the third impurity may be a toxic impurity, or a functional impurity, or an organic compound, or an inorganic compound, or bioburden or endotoxin, and the second impurity may be a toxic impurity, or a functional impurity, or an organic compound, or an inorganic compound, or bioburden or endotoxin. In a further example, at least one further impurity is meant to be understood as encompassing any number of further impurities, for example at least two further impurities, at least three further impurities, at least 4, at least 5, at least 6, at least 7, at least 8, at least 9, at least 10, at least 15, or at least 20 further impurities.

Therein, each indent may refer to a class of impurities as described in more detail above.

Advantageously, the method of the present disclosure may, for example, be used to even increase an impurity level in a cell culture medium. For example, such a measure may be particularly desirable in the case of a functional impurity, which - in contrast to the conventional use of the term "impurity" - may be required in a cell culture medium to ensure proper performance of the cell culture. Such a measure may, alternatively or additionally, be desirable in a case where an increase of an impurity level is carried out within an allowable range and/or to a level below an upper limit of the respective impurity. Such a measure may, alternatively or additionally, be desirable in a case where by increasing the impurity level of an impurity, the impurity level of a further impurity is reduced.

According to an exemplary embodiment of the method of the present disclosure, it may be determined that a particular functional impurity is to be added to the cell culture medium. This may for example be understood to mean that the functional impurity is added separately to the cell culture medium (e.g. added independently of a raw material in the cell culture medium). For example, it may be determined that the total impurity level of a functional impurity in the cell culture medium does not meet at least one predefined criterion by determining that the total impurity level of the functional impurity is below a predefined lower limit in the cell culture medium. In such an example, it may be determined that the functional impurity (e.g. a particular amount thereof) is to be added to the cell culture medium, such that by adding the functional impurity to the cell culture medium the predefined criterion is met.

According to this exemplary embodiment, the method of the present disclosure further comprises:

- determining, if it is determined that the total impurity level of a functional impurity in the cell culture medium does not meet the at least one predefined criterion, that the functional impurity is to be added to the cell culture medium; and, in particular,
- outputting information indicating that the functional impurity is to be added to the cell culture medium.

Advantageously, the method of the present disclosure may allow for optimizing a total impurity level even if no raw material with a suitable impurity profile is on stock.

According to an exemplary embodiment of the method of the present disclosure, the cell culture medium is of liquid form and wherein the formulation of the cell culture medium further indicates a hydration procedure for the plurality of raw materials.

For example, a hydration procedure is meant to be understood as a process of preparing a liquid cell culture medium at least partially based on a solid cell culture medium. Such a process may for example encompass the steps of adding at least one solvent (e.g. water), stirring and sterile filtration. Depending on the cell culture medium, a hydration procedure may for example encompass additional steps such as adjusting a pH value and/or adding additional substances.

Further considering the disclosed features above, the disclosure of a method step of the method of the present disclosure shall also be considered as a disclosure of an apparatus being configured to perform the respective method step and as a disclosure of an apparatus comprising means for performing the respective method step. Likewise, the disclosure of an apparatus being configured to perform a method step or the disclosure of an apparatus comprising means for performing a method step shall also be considered as a disclosure of the method step itself.

It is to be understood that the presentation of the embodiments disclosed herein is merely by way of examples and non-limiting.

Other features of the present disclosure will become apparent from the following detailed description considered in conjunction with the accompanying drawings. It is to be understood, however, that the drawings are designed solely for purposes of illustration and not as a definition of the limits of the present disclosure, for which reference should be made to the appended claims. It should be further understood that the drawings are not drawn to scale and that they are merely intended to conceptually illustrate the structures and procedures described herein.

**BRIEF DESCRIPTION OF THE FIGURES**

Some exemplary embodiments of the present disclosure will now be described with reference to the accompanying drawings.

| | |
|---|---|
| Fig. 1 | shows an exemplary environment in which the method according to the present disclosure may be performed; |
| Figs. 2a, 2b | show examples of a plurality of raw materials of a cell culture medium according to the present disclosure; |
| Fig. 3 | shows a flow chart illustrating an exemplary embodiment of the method according to the present disclosure; |
| Fig. 4 | shows a flow chart illustrating another exemplary embodiment of the method according to the present disclosure; |
| Fig. 5 | shows a flow chart illustrating another exemplary embodiment of the method according to the present disclosure; |
| Fig. 6 | shows a flow chart illustrating another exemplary embodiment of the method according to the present disclosure; |
| Figs. 7a to 7c | show another exemplary embodiment of the method according to the present disclosure; |
| Fig. 8 | shows a flow chart illustrating another exemplary embodiment of the method according to the present disclosure; |
| Fig. 9 | shows a scheme illustrating another exemplary embodiment of the method according to the present disclosure; |
| Figs. 10a, 10b | show exemplary experimental data according to the present disclosure; |
| Fig. 11 | shows further exemplary experimental data according to the present disclosure; |
| Fig. 12 | shows a schematic illustration of an exemplary embodiment of an apparatus according to the present disclosure; and |
| Fig. 13 | shows exemplary embodiments of storage media. |

**DETAILED DESCRIPTION OF THE FIGURES**

The following description serves to deepen the understanding of the present disclosure and shall be understood to complement and be read together with the description of exemplary embodiments of the present disclosure as provided in the above section of this description.

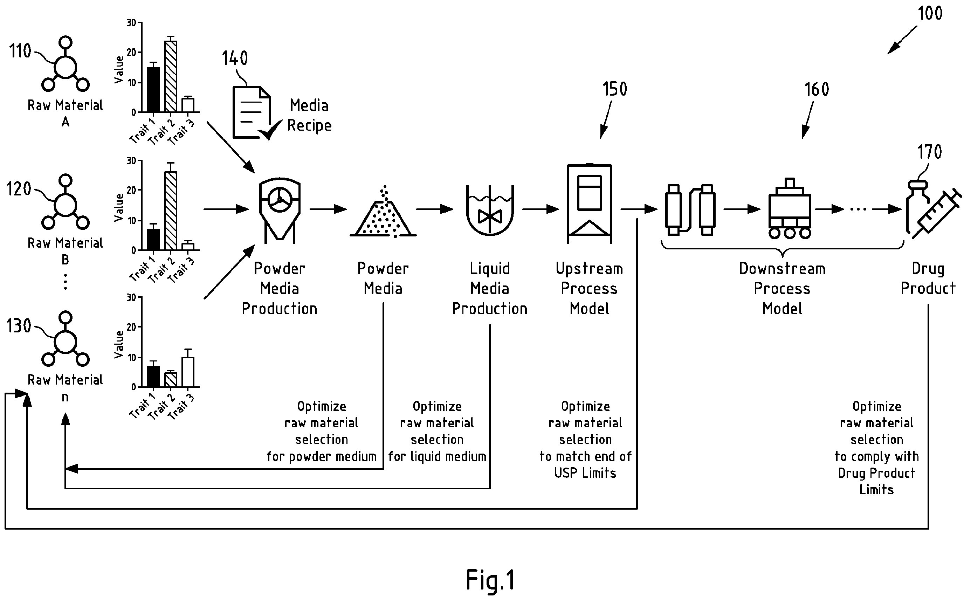

Fig. 1 shows an exemplary environment 100 in which the method according to the present disclosure may be performed.

To provide a general example, a cell culture medium may contain a plurality of different substances such as amino acids, amino acid derivatives, nucleotides, salts, biogenic amines, fats, sugars, vitamins, dyes or complex biological additives, each in a predefined amount. Therein, each of these substances may be separately sourced as what is called a raw material herein. The cell culture medium may be prepared as a powder which is required to be hydrated by a customer prior to use. Alternatively, the cell culture medium may, for example, be prepared in liquid form.

Without being limited thereto, a cell culture medium may contain at least one of the following raw materials: Biotin, Calcium Chloride ($CaCl_2$) (anhyd.), Choline chloride, Cupric sulfate ($CuSO_4 \cdot 5\,H_2O$), D-Calcium pantothenate, D-Glucose (Dextrose), Ferric Nitrate ($Fe(NO_3)_3 \cdot 9\,H_2O$), Ferric sulfate ($FeSO_4 \cdot 7\,H_2O$), Folic acid, Glycine, Hypoxanthine Na, i-Inositol, L-Alanine, L-Arginine hydrochloride, L-Asparagine $\cdot\,H_2O$, L-Aspartic acid, L-Cysteine hydrochloride $\cdot\,H_2O$, L-Cystine $\cdot$ 2 HCl, L-Glutamic acid, L-Glutamine, L-Histidine hydrochloride $\cdot\,H_2O$, Linoleic acid, Lipoic acid, L-Isoleucine, L-Leucine, L-Lysine hydrochloride, L-Methionine, L-Phenylalanine, L-Proline, L-Serine, L-Threonine, L-Tryptophan, L-Tyrosine disodium salt dihydrate, L-Valine, Magnesium chloride (anhyd.), Magnesium Sulfate ($MgSO_4$) (anhyd.), Niacinamide, Phenol Red, Potassium Chloride (KCl), Putrescine $\cdot$ 2 HCl, Pyridoxine hydrochloride, Riboflavin, Sodium Bicarbonate ($NaHCO_3$), Sodium Chloride (NaCl), Sodium phosphate dibasic ($Na_2HPO_4$) (anhyd.), Sodium Phosphate monobasic ($NaH_2PO_4 \cdot H_2O$), Sodium pyruvate, Thiamine hydrochloride, Thymidine, Vitamin B12, and/or Zinc sulfate ($ZnSO_4 \cdot 7\,H_2O$).

Without being limited thereto, a cell culture medium may contain at least one of the following raw materials:

Biotin, Calcium nitrate (Ca(NO$_3$)$_2$ · 4 H$_2$O), Choline chloride, D-Calcium pantothenate, D-Glucose (Dextrose), Folic acid, Glutathione (reduced), Glycine, i-Inositol, L-Arginine, L-Asparagine, L-Aspartic acid, L-Cystine · 2 HCl, L-Glutamic acid, L-Glutamine, L-Histidine, L-Hydroxyproline, L-Isoleucine, L-Leucine, L-Lysine hydrochloride, L-Methionine, L-Phenylalanine, L-Proline, L-Serine, L-Threonine, L-Tryptophan, L-Tyrosine disodium salt dihydrate, L-Valine, Magnesium Sulfate (MgSO$_4$) (anhyd.), Niacinamide, Para-aminobenzoic acid, Phenol Red, Potassium Chloride (KCl), Pyridoxine hydrochlo-ride, Riboflavin, Sodium Bicarbonate (NaHCO$_3$), Sodium Chloride (NaCl), Sodium phosphate dibasic (Na$_2$HPO$_4$) (an-hyd.), Thiamine hydrochloride, and/or Vitamin B12.

Further examples of raw materials for cell culture media are known in the field, as for example evidenced by the prior patent applications WO 2007/036291 A2 and WO 2009/087087 A1, which are incorporated herein in their entirety.

Due to the manufacturing process of the raw materials, beside the intended substance, each raw material may comprise one or more impurities such as one or more toxic substances, in particular elements and/or compounds, one or more functional substances, in particular elements and/or compounds, and/or bioburden, and/or endotoxin. In some examples, impurities may be understood to be of non-animal origin. These impurities end up in the cell culture media prepared from the respective raw material. For example, exceeding a certain limit amount of a given impurity in a cell culture medium may lead to undesired effects such as reduced product yield, impaired product quality or even a contaminated cell culture product, which is potentially unusable or undesired in a downstream application such as for example human therapeutic applications. As a consequence, manufacturers of cell culture media aim to only use those raw materials which avoid impurities and any of such undesired effects.

For example, in the context of some cell culture products such as biotherapeutics, strict regulations exist with respect to the physical, chemical, biological, or microbiological characteristics, which should be controlled within predefined limits to ensure the safety, efficacy, and quality of the product In the field, these so-called critical quality attributes (CQA) are generally considered essential for the development, manufacturing, and regulatory approval of cell culture products such as biotherapeutic drugs. The cell culture medium may, for example, affect CQA of the product such as purity, post-translational modifications, protein folding and disulfide bond formation, and/or safety with respect to administering the product to a patient. In order to provide a pure, high-quality and safe cell culture product, the cell culturing process must be carried out in a cell culture medium that does not introduce harmful contaminants and/or impurities into the cell culture or the finished drug product itself.

For example, known guidelines such as the EMA scientific guideline ICH Q3D provide limit amounts for certain elemental impurities in finished drug products. Beyond that, some non-binding estimations - such as for example based on literature - may exist that correlate the ICH Q3D limits to limits of elemental impurities in the raw materials of cell culture media, taking into account dilution effects in downstream processing steps. These estimations may have the disadvantage of being unprecise, for example because limits being derived from finished drug product legislations (e.g. ICHQ3D) are not specifically tailored for such use. Accordingly, it may be challenging to identify from a plurality of raw materials the one raw material responsible for an undesired effect caused by the cell culture medium. Moreover, applying the rules of, for example, scientific guidelines relating to the raw materials instead of a drug product may lead to non-use of a specific raw material or batch thereof, which potentially leads to unnecessary waste and increased costs both for the manufacturer and the final customer of the cell culture medium.

Due to the sheer number of raw materials in a given cell culture medium and the number of batches usually present at the manufacturing site for each raw material, it may be challenging to calculate the resulting impurity levels for every possible combination of batches in a given cell culture medium.

Therefore, there exists a need to provide a reliable, fast and accurate method to calculate the respective amounts of impurities in a cell culture medium. Preferably, the method according to the present disclosure shall be set up to detect those raw materials that need to be altered or exchanged in order to improve (e.g. reduce or increase) the respective amounts of the respective impurities in a cell culture medium. Still preferably, the method shall allow the use of raw materials having an allowable range of impurity levels to enable robust, flexible and economical raw material sourcing by allowing the impurity levels to vary within allowable ranges. Still preferably, the method shall allow to determine a cell culture medium or a portfolio of cell culture media complying with regulations and/or CQA requirements.

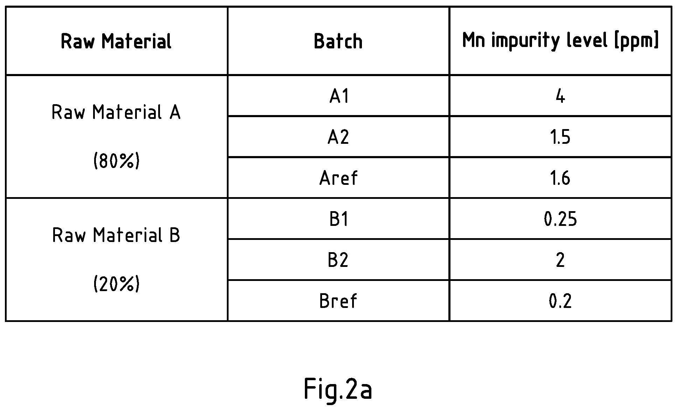

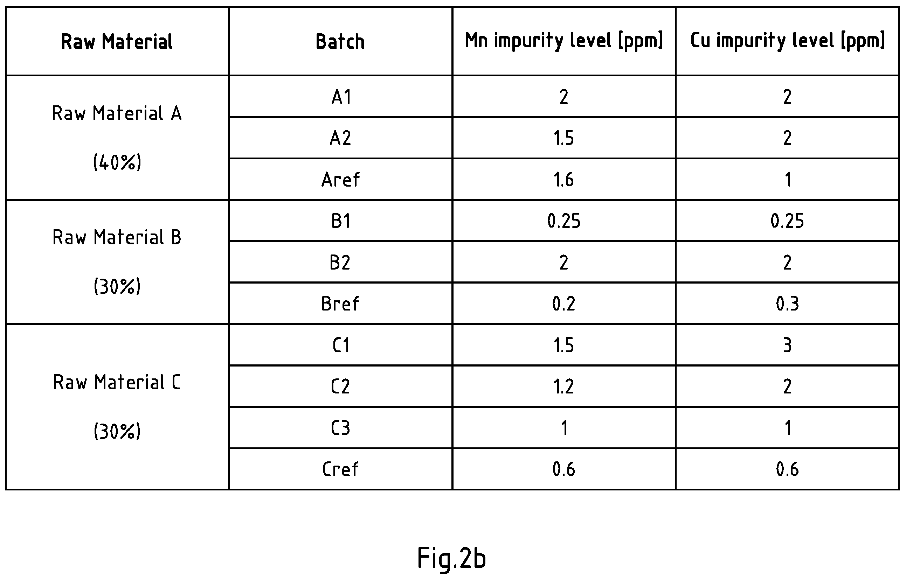

With reference to the non-limiting example of Fig. 1 and according to the method of the present disclosure, a cell culture medium to be optimized including a plurality of $n$ raw materials 110, 120, 130 (see raw materials A, B, ..., n) may be considered. Therein, a formulation 140 (see "Media Recipe") indicating a ratio of the plurality of raw materials in the cell culture medium may be obtained (e.g. 40 % of raw material A, 30 % of raw material B etc. as described below with reference to Fig. 2a and Fig. 2b). Further, respective impurity levels of at least one impurity in each raw material of the plurality of $n$ raw materials may be obtained. In the example shown in Fig. 1, three impurities (see "Trait 1, "Trait 2" and "Trait 3" in Fig. 1) are considered, wherein respective impurity levels (see the values of "Trait 1, "Trait 2" and "Trait 3" in Fig. 1) of these three impurities in each raw material of the plurality of $n$ raw materials are obtained.

Further referring to the example shown in Fig. 1, it may be assumed that a cell culture medium based on the plurality of $n$ raw materials 110, 120, 130 and the corresponding formulation 140 is produced. Therein, the method of the present disclosure comprises determining, at least partially based on the formulation 140 (see "media recipe" in Fig.

1) and on the respective impurity levels (see the values of "Trait 1, "Trait 2" and "Trait 3" in Fig. 1), respective total impurity levels of the three impurities in the cell culture medium. According to the method of the present disclosure, it may for example be considered whether the cell culture medium is of powder form (see "Powder Media" in Fig. 1) or liquid form (see "Liquid Media Production" in Fig. 1). In the latter case, the formulation 140 of the cell culture medium may indicate a hydration procedure for the plurality of *n* raw materials 110, 120, 130, which for example encompasses the steps of adding at least one solvent (e.g. water) to the cell culture medium in powder form, stirring and sterile filtration.

As indicated in Fig. 1, determining, at least partially based on the formulation 140 and on the respective impurity levels, a total impurity level of the at least one impurity in the cell culture medium may be used to optimize the raw material selection (e.g., for cell culture media in powder form or liquid form). Optimizing the raw material selection may comprise various steps as described by the following examples.

In one example, a respective contribution of one or more raw materials of the plurality of *n* raw materials to the total impurity levels of the three impurities shown in Fig. 1 may be determined, which may for example comprise determining a sensitivity matrix as further described below with reference to Figs. 2a and 2b. For example, based on the rather high level of the first impurity in raw material A (see "Trait 1" for raw material A in Fig. 1) and the content of raw material A in the cell culture medium according to the corresponding formulation, it may be determined that raw material A has a larger contribution to the total impurity level of the first impurity than the other raw materials. For example, it may follow from this that the cell culture medium is particularly sensitive to the exact impurity level in raw material A, which therefore should for example be kept constant (e.g. constant within a predefined range) when used for media production.

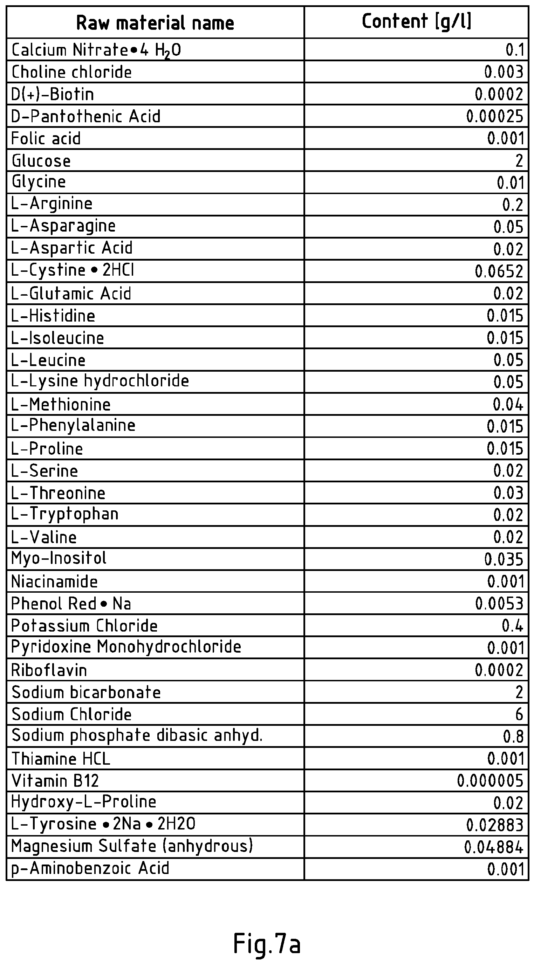

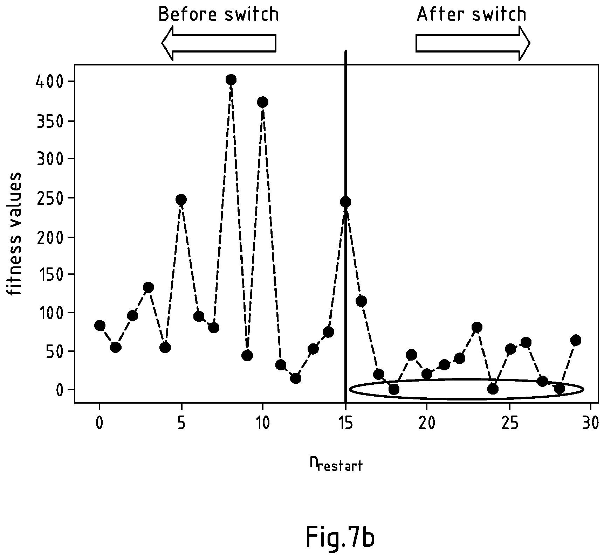

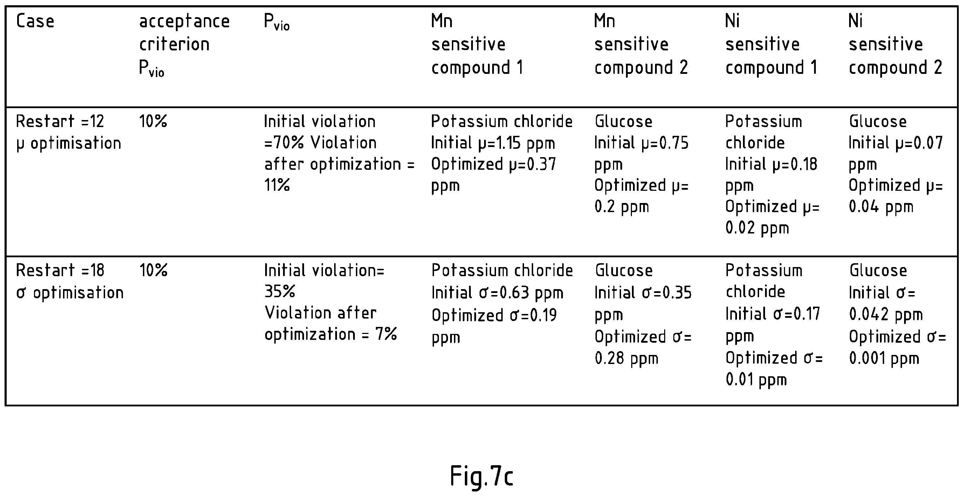

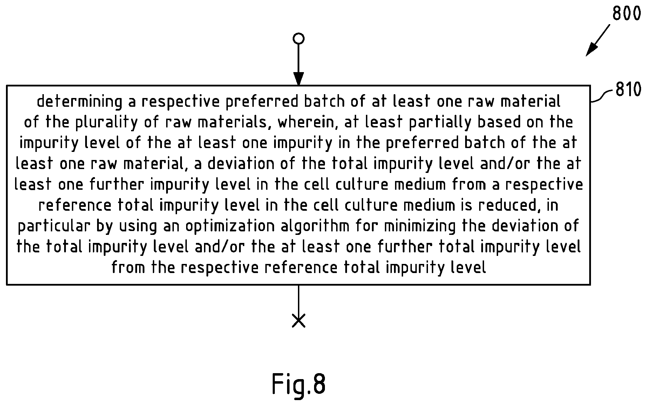

In another example, it may be determined whether the total impurity levels of the three impurities in the cell culture medium meet at least one predefined criterion. This may for example comprise determining whether a particular total impurity level may be below a predefined upper limit in the cell culture medium (e.g. considering that this particular impurity level relates to a toxic impurity) and/or determining whether a particular total impurity level may be above a predefined lower limit in the cell culture medium (e.g. considering that this particular impurity level relates to a functional impurity). In a further example, a deviation of a particular total impurity level in the cell culture medium from a respective reference total impurity level in the cell culture medium may be determined, wherein such a deviation may be reduced by determining a preferred batch of at least one raw material of the plurality of *n* raw materials as further described below with reference to Figs. 7 and 8.

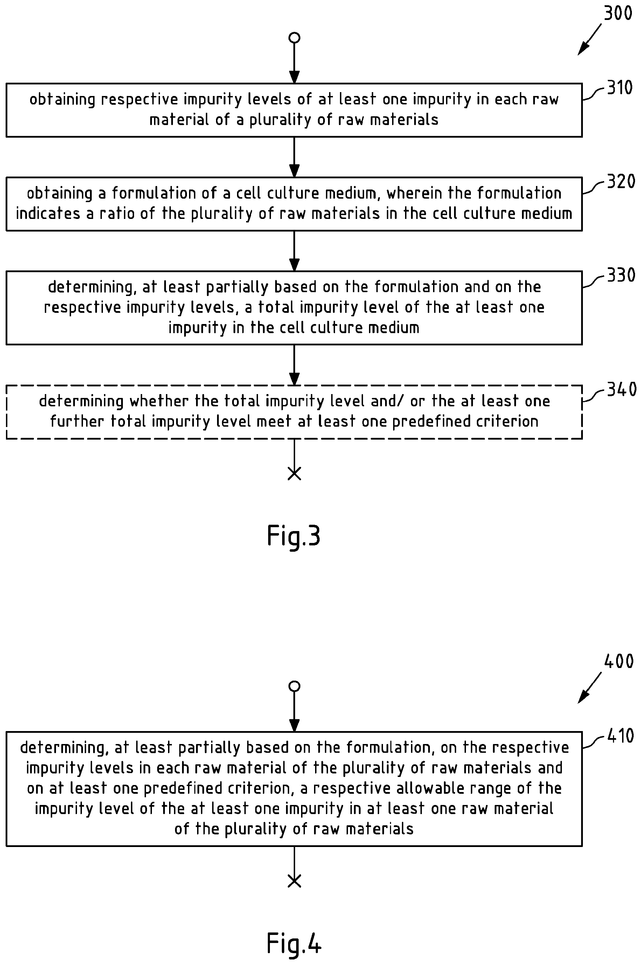

In another example, a respective allowable range of a particular impurity level in at least one of the raw materials of the plurality of *n* raw materials may be determined. For example, it may be determined that the impurity level of the first impurity in raw material A (see "Trait 1" for raw material A in Fig. 1) may vary within an allowable range, while the total impurity level of this first impurity in the cell culture medium still meets a predefined criterion (e.g. an upper and/or lower limit). Further details on determining an allowable range are described with reference to Figs. 4 to 6.

Further referring to the example shown in Fig. 1, the method according to the present disclosure may further consider an upstream process model 150 and a downstream process model 160 of the cell culture medium. Thereby, optimizing the raw material selection may not only be possible for the cell culture media production, but also with regard to the upstream and downstream process following the cell culture media production as indicated in Fig 1. For example, an upstream process may refer to earlier stages of a production process, where cells (e.g., mammalian cells) may, for example, be genetically modified to produce a desired protein (e.g., a therapeutic antibody). For example, this phase may typically include activities such as cell culture media optimization, cell line development, genetic engineering and/or cell banking. The downstream process, in another example, may refer to the later stages of the production process, where for example a protein produced may be purified and prepared for use. For example, this phase may typically include activities such as cell harvesting, chromatography, filtration and/or formulation.

In a general example, the drug product 170 as end result of the process indicated in Fig. 1 may be regulated by health authorities, such that optimizing the raw material selection may be improved by not only determining total impurity levels in the cell culture medium, but also potential changes to such impurity levels introduced during the upstream and downstream process. This additional consideration of the upstream and downstream process may in some examples provide more lenient limits for the total impurity levels in the cell culture medium and thus for the impurity levels in the raw materials. For example, modelling the downstream process may be used as an additional tool to perform raw material selection due to certain impurity limits not encompassed during cell culture media optimization or upstream processing. Further details on considering an upstream process model and downstream process model are described with reference to Fig. 3.

Several further advantages may arise from an optimized raw material selection according to the method of the present disclosure as exemplarily illustrated in Fig. 1. For example, depending on a given application and predefined criteria, the most suitable raw materials may be selected. Further, it may for example be possible to determine key impurities that vitally affect the performance of the resulting cell culture medium. Setting allowable ranges (e.g., by defining allowable boundaries) for these key impurities may allow for more robust sourcing of raw materials and for minimizing batch failure rates. In further examples, by setting allowable ranges only for key impurities and/or only for highly influential raw materials, the proposed method may lead to less strict limits in terms of impurity content of raw material batches, which in turn may increase supply chain robustness and flexibility, which may also lead to a decrease in costs due to fewer special small batches of raw materials needed.

Figs. 2a and 2b show examples of a plurality of raw materials of a cell culture medium according to the present disclosure.

In one example, the method according to the present disclosure may be used to optimize the composition of raw materials in a cell culture medium comprising two raw materials. For each of the plurality of two raw materials termed raw material A and raw material B, two batches are available. In the following, batch 1 of raw material A will be referred to as "A1", batch 2 of raw material A will be referred to as "A2", batch 1 of raw material B will be referred to as "B1", and batch 2 of raw material B will be referred to as "B2". Additionally, for each of the raw materials, a reference batch relating to a commercially available cell culture medium may be available, which in the following will be referred to as "Aref" and "Bref" for raw materials A and B, respectively. Impurity levels from such reference batches may for example be determined by measurement or by estimation.

For each of the batches A1, A2, B1, B2, Aref, Bref, the impurity level of the functional impurity manganese (Mn) is obtained (e.g. by measuring or by retrieving the impurity levels from a database) as shown in the table of Fig. 2a. For the cell culture medium, a formulation is obtained indicating that the cell culture medium consists of 80 % raw material A and 20 % raw material B. From these exemplary data, it is determined that a cell culture medium consisting of raw materials from the batches Aref and Brefwould contain a total Mn impurity level of 1.32 ppm (=[0.8*1.6 ppm]+[0.2*0.2ppm]), which may be referred to as the reference total impurity level of Mn in the cell culture medium. For batches A1, A2, B1, B2, the deviation of the total Mn impurity level from the reference total impurity level is determined, and additionally, it is determined whether the total Mn impurity level is below an upper limit.

Based on these exemplary data, a composition of the cell culture medium having a reduced deviation from the reference total Mn impurity level is determined by means of an optimization algorithm. It may for example be determined that a cell culture medium consisting of 80 % A2 and 20 % B1 has a reduced deviation from the reference total Mn impurity level, having a total Mn impurity level of 1.25 ppm. In contrast, for example a cell culture medium consisting of 80 % A1 and 20 % B2 has a total Mn impurity level of 3.60 ppm, which constitutes a larger deviation from the reference total Mn impurity level compared to 1.25 ppm, and in addition is above the upper limit of 1.32 ppm. Thus, batches A2 and B1 may for example be determined as preferred batches of raw materials A and B, respectively, for the cell culture medium.

In another example, the method according to the present disclosure may be used to optimize the composition of raw materials in a cell culture medium comprising the plurality of three raw materials A, B, and C. For raw materials A and B, two batches each are available (referred to as batches A1, A2, B1, B2, respectively, similar to Fig. 2a). For raw material C, three batches are available, referred to as C1, C2, C3. Additionally, for each of the raw materials A, B, and C, a reference batch relating to a commercially available cell culture medium may be available, referred to as "Aref", "Bref", and "Cref", respectively.

For each of the batches A1, A2, B1, B2, C1, C2, C3, Aref, Bref, and Cref, the impurity level of the functional impurity manganese (Mn) and the impurity level of one further impurity Copper (Cu) is obtained (e.g. by measuring and/or by retrieving the impurity levels from a database) as shown in Fig. 2b. For the cell culture medium, a formulation is obtained indicating that the cell culture medium consists of 40 % raw material A, 30 % raw material B, and 30 % raw material C. It may be assumed as an example that the impurities are weighted as follows (e.g. regarding their functional importance): Mn 0.7, Cu 0.3. From these exemplary data, it is determined that a reference cell culture medium consisting of raw materials from the reference batches Aref, Bref and Cref contains a reference total Mn impurity level of 0.88 ppm and a reference total Cu impurity level of 0.67 ppm, which results in a reference total weighted impurity level of 0.817 ppm.

Based on these exemplary data, a composition of the cell culture medium having a reduced (e.g. a minimum) deviation from the reference total Mn and Cu impurity levels is determined (e.g. by means of an optimization algorithm). It may for example be determined that a cell culture medium consisting of 40 % A2, 30 % B1 and 30 % C3 has the reduced deviation from the reference total Mn and Cu impurity levels, having a total Mn impurity level of 0.975 ppm and a total Cu impurity level of 1.175 ppm, which results in a total impurity level of 1.035 ppm. In contrast, for example a cell culture medium consisting of 40 % A1, 30 % B2 and 30 % C1 has a total impurity level of 1.99 ppm, which constitutes a larger deviation from the reference total impurity level of 0.817 ppm. Thus, batches A2, B1, and C3 may be determined as preferred batches of raw materials A, B, and C, respectively, for the cell culture medium.

Compared to the exemplary data sets in Fig. 2a und 2b, cell culture media with a significantly larger number of raw materials, impurities and batches may be input to the method according to the present invention. In such cases, it may be advantageous to determine a respective contribution of one or more raw materials to a particular total impurity level and subsequently consider only those raw materials whose contribution to the total impurity is larger than a predefined threshold.

In a non-limiting example, only those impurities (denoted "traits" in the following examples) $k$ of a raw material $i$ may be considered for optimizing a cell culture medium that contribute at least a certain minimum percentage to the total impurity level in a target medium $m$. This may for example be expressed by elements $s_{i,k}^m$ of a sensitivity matrix $S^m$ for a medium $m$, wherein $S^m \in \mathbb{R}^{n_{\text{Raw Materials}} \cdot n_{\text{Traits}}}$ :

$$s_{i,k}^m = \frac{\text{Trait}_{i,k} \cdot y_{i,m}}{\text{Trait}_{m,k}} \geq \beta_{k,m},$$

wherein $y_{i,m}$ denotes the mass fraction of the raw material $i$ in medium $m$ for powder formulations and

$\text{Trait}_{m,k} = \sum_{i=1}^{n_{\text{Raw Materials}}} \text{Trait}_{i,k} \cdot y_{i,m}$ with $\text{Trait}_{i,k} = \mu_{i,k}$. Here, $\mu_{i,k}$ denotes the average value of impurity trait $k$ of raw material $i$, where without limitation the average has been determined from a representative set of reference batches of the raw material. For example, the predefined threshold $\beta_{k,m}$ may be set to 0.05. In examples of liquid formulations, the summation may run over an extended set of materials $n_{\text{Raw Materials}} + n_{\text{Liquid Prep Supplements}}$ and $\text{Trait}_{i,k} \cdot x_{i,m}$ replaces $\text{Trait}_{i,k} \cdot y_{i,m}$, wherein $x_{i,m}$ is the corresponding liquid media mass fraction. In further examples of optimizing a whole portfolio of cell culture media, the respective raw material may for example be considered if it fulfils the above criterion for at least one of the media in the portfolio. This approach may for example ensure that as few raw materials as possible are endowed with specification limits that pose additional effort in the sourcing process for production.

Regarding a deviation between a reference total impurity level and a determined total impurity level as for example described above with reference to Fig. 2a and Fig. 2b, a non-limiting example for comparing these total impurity levels (denoted "traits" in the following example) shall be given in the following.

The following formula may be used to express a deviation between a determined total impurity (e.g. referred to as simulated trait) and a total reference impurity level (e.g. referred to as reference trait):

$$f_{\text{obj}}^{\text{Media A}} = \sum_{i=1}^{n} w_i \left( \left( \text{Trait}_i^{\text{Media A,Ref}} - \text{Trait}_i^{\text{Media A,Sim}} \right)^2 \right) \Big/ \left( \text{Trait}_i^{\text{Media A,Ref}} \right)^2.$$

For example, the division by $\left( \text{Trait}_i^{\text{Media A,Ref}} \right)^2$ may be optional when determining a relative error sum. In a further example, the value of the objective function $f_{\text{obj}}^{\text{Media A}}$ for a given cell culture medium (e.g. media A) may be optimized (e.g. maximized or minimized). For example, for each of the $n$ impurities to be analyzed, the deviation between the impurity level in a reference medium, $\text{Trait}_i^{\text{Media A,Ref}}$, which may for example be an impurity level of said impurity in a reference medium or an upper and/or lower limit of said impurity level - and the impurity level of said impurity in a simulated or respective calculated cell culture medium, $\text{Trait}_i^{\text{Media A,Sim}}$, is calculated and squared to generate an absolute error sum for said impurity. For example, each impurity may be weighted by multiplying the absolute error sum with a weighting factor $w_i$. The absolute error sums, which are optionally weighted, of all impurities to be analyzed may then be added to generate the value of the objective function $f_{\text{obj}}^{\text{Media A}}$.

For example, based on the exemplary objective function given above, the method of the present disclosure aims for minimizing the value of $f_{\text{obj}}^{\text{Media A}}$ when $\text{Trait}_i^{\text{Media A,Ref}}$ corresponds to the impurity level of an impurity $i$ in a reference medium or for maximizing the value of $f_{\text{obj}}^{\text{Media A}}$ when $\text{Trait}_i^{\text{Media A,Ref}}$ corresponds to a predetermined upper and/or lower limit of the impurity level of an impurity i in a reference medium (e.g. for maximizing a distance to the upper and/or lower limit).

For example, for calculating the value of $\mathrm{Trait}_i^{\mathrm{Media\,A,Sim}}$ in the exemplary objective function given above, the following formula may be used:

$$\mathrm{Trait}_i^{\mathrm{Media\,A,Sim}} = \mathrm{Trait}_{i,j,k}^{\mathrm{Media\,A}} = \sum_{j=1}^{n\,\mathrm{RM}} w_{ji} \cdot \mathrm{Trait}^{\mathrm{RM}_j} \cdot F(k).$$

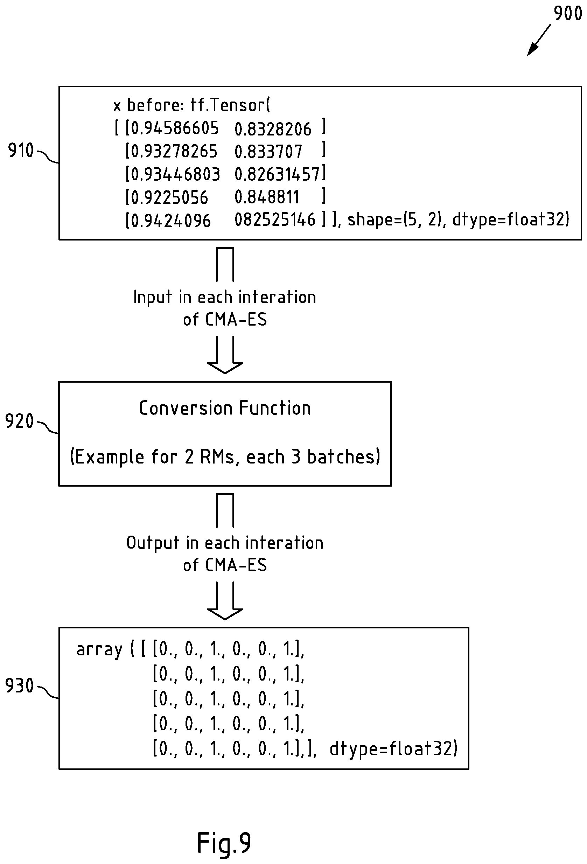

According to this exemplary formula, the total impurity level $\mathrm{Trait}_i^{\mathrm{Media\,A,Sim}}$ of a particular impurity $i$ in a simulated cell culture medium is equal to the sum of impurity levels of this particular impurity $i$ in the plurality of raw materials $n$ included in the medium. In further detail, not only respective impurity levels in the plurality of $n$ raw materials may be considered, but further respective impurity levels in different batches of each raw material. For including or not including a batch of a particular raw material in the cell culture medium to be optimized, $k$ in $F(k)$ may be 0 (i.e. the batch is not included) or 1 (i.e. the batch is included). For example, when minimizing the objective function, a preferred batch of each raw material with a respective impurity level may be determined, as it will be further described with reference to Figs. 8 and 9.

For example, based on the exemplary objective function given above, the method of the present disclosure may also be applied to not only optimize a selection of raw materials with respect to one or more impurities in a single cell culture medium, but it may further be applied to optimize a plurality of cell culture media (e.g. a media portfolio) with respective media formulations. For example, the objective functions of $n$ cell culture media may be jointly optimized according to:

$$f_{\mathrm{obj}}^{\mathrm{Media\,Portfolio}} = \sum_{i=1}^{n\,Media} w_x \cdot f_{\mathrm{obj}}^x,$$

wherein the factor $w_x$ may represent an optional weighting of a particular medium within the portfolio.

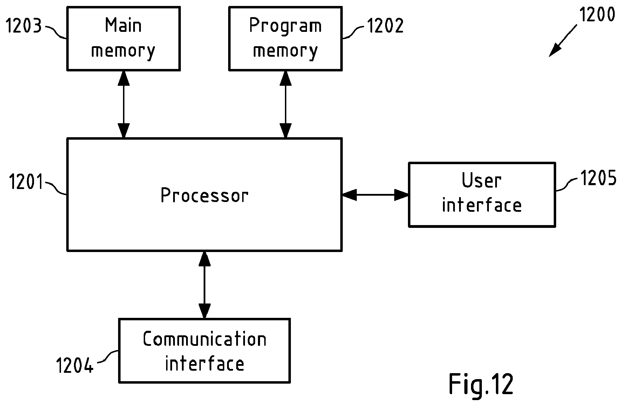

Fig. 3 shows a flow chart 300 illustrating an exemplary embodiment of the method according to the present disclosure. Without limiting the scope of the present disclosure, it may be assumed that the actions 310 to 340 of flow chart 300 may be performed by an apparatus as described with reference to Fig. 12.

Action 310 is obtaining respective impurity levels of at least one impurity in each raw material of a plurality of raw materials.

Considering the exemplary pluralities of raw materials described with reference to Figs. 2a and 2b, respective impurity levels of the impurity Mn in each raw materials A, raw material B and raw material C are obtained. Further, respective impurity levels of at least one further impurity Cu in each raw material A, raw material B and raw material C are obtained. Obtaining these impurity levels may for example comprise retrieving the impurity levels from a raw material database or causing (e.g. by instructing and/or controlling) measuring at least one of these impurity levels. Considering for example the apparatus shown in Fig. 12 below performing action 310, the apparatus communication interface may be used for obtaining respective impurity levels (e.g. by obtaining the respective impurity levels from another apparatus such as a database apparatus or a measurement apparatus).

Action 320 is obtaining a formulation of a cell culture medium, wherein the formulation indicates a ratio of the plurality of raw materials in the cell culture medium.

Considering the examples described with reference to Figs. 2a and 2b, a formulation of a cell culture medium is obtained, wherein the formulation may indicate that the plurality of two raw materials A and B are contained in the cell culture medium in a ratio of 80 % to 20 % (see Fig. 2a). In another example, the formulation may indicate that the plurality of three raw materials A, B and C are contained in the cell culture medium in a ratio of 40 % to 30 % to 30 %. For example, obtaining a formulation of a cell culture medium may comprise retrieving the formulation from a media formulation database.

Action 330 is determining, at least partially based on the formulation and on the respective impurity levels, a total impurity level of the at least one impurity in the cell culture medium.

Considering the examples described with reference to Figs. 2a and 2b, a total impurity level of the impurity Mn and/or at least one further total impurity level of the impurity Cu in the cell culture medium produced according to the corresponding formulation may be determined based on the respective impurity levels in each raw material. Therein, the respective impurity levels in each raw material may refer to a respective batch of the respective raw material (e.g. batches A1, A2 of raw material A or e.g. batches B1, B2 of raw material B).

In another non-limiting example of action 330, a respective total impurity level $w^{tot}$ of an impurity $k$ in a cell culture medium $j$ of powder form including a plurality of $i = 1$ to $n$ raw materials may be determined according to:

$$w_{k,j}^{tot} = \sum_i w_{k,i} \cdot w_i.$$

In another non-limiting example of action 330, a respective total impurity level $c^{tot}$ of an impurity $k$ in a cell culture medium $j$ of liquid form including a plurality of $i$ = 1 to $n$ raw materials may be determined according to:

$$c_{k,j}^{tot} = c_j \cdot \sum_i w_{k,i} \cdot w_i.$$

In these examples, $w_{k,i}$ is a mass fraction (e.g. the impurity level) of impurity $k$ in raw material $i$, $w_i$ is mass fraction of raw material $i$ in medium $j$ (as e.g. indicated by a formulation of the medium $j$) and $c_j$ is a concentration of medium powder $j$ in the liquid medium (e.g. specified in g/L, as further indicated by a formulation of medium $j$).

For example, information indicating the respective total impurity levels of Mn and/or Cu may be output as outcome of action 330. Considering for example the apparatus shown in Fig. 12 performing action 330, the apparatus user interface may be used for outputting respective total impurity levels (e.g. by presenting the total impurity levels on a screen as visual user output).

In one example, a respective contribution of the raw materials referred to in Figs. 2a and 2b to the total impurity levels of Mn and/or Cu may be determined, which may for example comprise determining a sensitivity matrix as further described with reference to Figs. 2a and 2b.

Optional action 340 is determining whether the total impurity level and/or the at least one further total impurity meet at least one predefined criterion. For example, action 340 may comprise determining whether the total impurity levels and/or the at least one further total impurity level are below a predefined upper limit in the cell culture medium and/or determining whether the total impurity level and/or the at least one further total impurity level are above a predefined lower limit in the cell culture medium.

Considering the examples described with reference to Figs. 2a and 2b, it may be determined whether the total impurity levels of Mn and/or Cu in the respective cell culture medium is below a predefined upper limit and/or above a predefined lower limit for the respective impurity in the respective cell culture medium (e.g. by comparing the determined total impurity levels with the respective upper and/or lower limit).

In a non-limiting example, it may be assumed that at least one total impurity level of a toxic impurity and at least one total impurity level of a functional impurity is determined in action 330 and that for both these impurities it is determined in action 340 whether a predefined criterion is met. In such an example, action 340 may comprise determining whether the total impurity level of the toxic impurity is below a predefined upper limit and whether the total impurity level of the functional impurity is above a predefined lower limit and below a predefined upper limit.

In another non-limiting example of optional action 340, determining whether the total impurity level and/or the at least one further total impurity level meet at least one predefined criterion is at least partially based on an upstream process model and/or on a downstream process model of the cell culture medium. For example, the determined respective total impurity levels in the cell culture medium may then be multiplied with additional factors representing the downstream process before determining whether the respective total impurity levels meet at least one predefined criterion (e.g. an upper and/or lower limit). For example, such additional factors according to a downstream process model may be considered together with a weighted sum of different media according to an upstream process model.

Further considering for example an upstream process model, a scenario may be assumed for a total impurity level of a particular impurity in the cell culture medium, where during the upstream process any compound uptake of the impurity by cells in the bioprocess would be explicitly neglected (e.g., this may be achieved based on a standard mass balancing approach).

Further, any possible dilution of the impurity (e.g. due to addition of acid, base or antifoam) during the upstream process may be neglected. For example, a mixture of media (considering e.g. a fed batch process with a basal media and feed media, e.g. according to a weighted combination of these media) may be considered, such that the total impurity level $\text{Trait}_{k,\text{USP}}^{\text{end}}$ after an upstream process including $k$ subprocesses may be determined.

Considering for example a downstream process model, an expected dilution of a particular impurity in a cell culture medium may be taken into account. Since most downstream processing steps may for example lead to a dilution of an impurity, the level of this impurity in the final drug product may be usually smaller compared to the total impurity level in the cell culture medium. Accordingly, more relaxed upper and/or lower limits may be assumed for a total impurity level in the cell culture medium. For example, the impurity level ("trait") after considering an upstream and downstream model process may be expressed as:

$$\text{Trait}_{k,\text{DSP}}^{\text{end}} = \text{Trait}_{k,\text{USP}}^{\text{end}} \cdot \prod_{l=1}^{n_{\text{Process steps}}} \frac{\text{Yield}_k^l}{\text{Dilution}_k^l},$$

wherein $l$ = 1 to n subprocesses included in the downstream process are considered.

Fig. 4 shows a flow chart 400 illustrating another exemplary embodiment of the method according to the present disclosure. For example, action 410 may be performed after action 330 or after action 340 as described with reference to Fig. 3. Without limiting the scope of the present disclosure, it may be assumed that action 410 of flow chart 400 may be performed by an apparatus as described with reference to Fig. 12.

Action 410 is determining, at least partially based on the formulation (e.g. the formulation obtained in action 320), on the respective impurity levels of the at least one impurity in each raw material of the plurality of raw materials (e.g. the impurity levels obtained in action 310) and on at least one predefined criterion (see action 340), a respective allowable range of the impurity level of the at least one impurity in at least one raw material of the plurality of raw materials. Therein, an allowable range of the impurity level of an impurity in a raw material may for example be understood as a numerical range of the impurity level within which the impurity level of this impurity in the raw material may be varied, while the total impurity level of this impurity in the cell culture medium still meets the predefined criterion (e.g. an upper and/or lower limit). In other words, a total impurity level of the at least one impurity in the cell culture medium, determined at least partially based on the formulation and on an (e.g. any) impurity level of the at least one impurity in the at least one raw material within the determined allowable range, meets the at least one predefined criterion (e.g. meets the at least one predefined criterion with a particular probability).

In a non-limiting example of action 410, determining a respective allowable range of an impurity level of at least one impurity in at least one raw material of the plurality of raw materials may not be based on the respective impurity levels of the at least one impurity in each raw material of the plurality of raw materials, but on the respective impurity levels in those one or more raw materials whose contribution to the total impurity level is larger than a predefined threshold. For example, this may reduce the optimization effort when determining the respective allowable range.

Considering the example described with reference to Fig. 2a, based on the formulation of a corresponding cell culture medium including raw materials A and B, the respective impurity levels of the impurity Mn in each raw material A and B (e.g. referring to respective batches of raw materials A and B, respectively) and a predefined criterion according to which the total impurity level of Mn in the cell culture medium should be above a lower limit and/or below an upper limit, a respective allowable range (e.g. +/- 20 % around the total impurity level of Mn in a reference cell culture medium which may e.g. consist of batches Aref and Bref for raw materials A and B, respectively) may be determined within which the impurity level of Mn in a raw material may vary, while the predefined criterion of the full cell culture medium is still met. Further, such an allowable range may be additionally determined for the impurity level of Mn in raw material B and/or may be additional determined for further total impurity levels (e.g. the impurity levels of Mn and Cu in the raw materials A, B and C of the cell culture medium referred to in Fig. 2b).

In another non-limiting example of action 410, determining the respective allowable range of the impurity level of the at least one impurity in at least one raw material of the plurality of raw materials may comprise calculating, at least partially based on an initial range of the impurity level of the at least one impurity in the at least one raw material, a probability that the total impurity level of the at least one impurity in the cell culture medium fails the at least one predefined criterion and determining, in particular by using an optimization algorithm, the respective allowable range of the impurity level based on the calculated failure probability and the initial range of the impurity level.

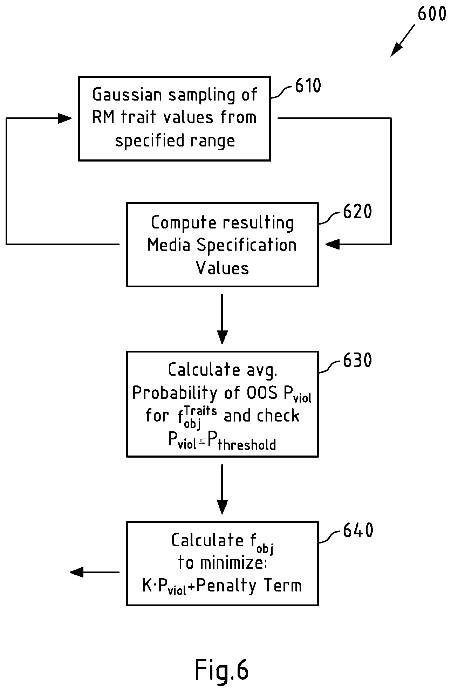

Considering the examples described with reference to Fig. 2a, an initial range of the impurity level of Mn in raw material A may be assumed (e.g. +/- 20 % around the impurity level Aref of Mn in raw material A), wherein such an initial range may for example be based on statistical variations of this impurity level within raw material A (e.g. by considering a plurality of batches of raw material A). Subsequently, a probability $p_{\text{viol}}$ (e.g. an out-of-specification probability for the target medium) may be determined that the total impurity level of Mn in the cell culture medium may fail the at least one predefined criterion (e.g. that a deviation of the determined total impurity level of Mn from a reference total impurity level of Mn in the cell culture medium is larger than an upper limit). This probability $p_{\text{viol}}$ may be compared to a threshold probability, and the initial range may be decreased if $p_{\text{viol}}$ is larger than the threshold probability to determine the allowable range. In another example, the initial range may be increased if $p_{\text{viol}}$ is lower than the threshold probability to determine the allowable range (e.g., $p_{\text{viol}}$ may then indicate a violation probability of the cell culture medium impurity level staying within an accepted two-sided trait band). Such an adaption of the initial range may for example be repeated for a plurality of iterations as part of an optimization algorithm to determine the allowable range (see Fig. 6 below).

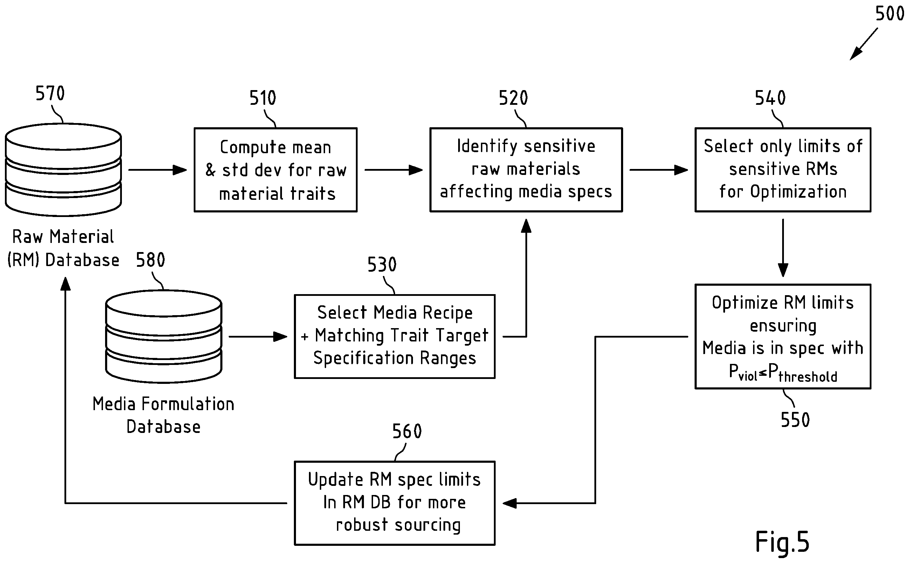

Fig. 5 shows a flow chart 500 illustrating another exemplary embodiment of the method according to the present disclosure. For example, the actions of flow chart 500 may be performed after action 330 or after action 340 as described with reference to Fig. 3 or as part of action 410 as described with reference to Fig. 4. Without limiting the scope of the present disclosure, it may be assumed that the actions of flow chart 500 may be performed by an apparatus as described with reference to Fig. 12.

In a first action 510, mean values and standard deviation values for particular impurity levels of respective impurities in raw materials ("raw material traits") may be computed based on a raw material database 570. These computed values may be used to determine initial ranges for impurity levels of respective impurities in each raw material. Subsequently, those raw materials out of a plurality of raw materials ("sensitive raw materials") included in a cell culture medium may be identified that have a particular contribution to the total impurity levels of interest in the cell culture medium (see action 520, e.g. by means of a sensitivity matrix as described with reference to Figs. 2a and 2b). This identification of sensitive raw materials may be based on the corresponding formulation of the cell culture medium ("media recipe"), which may be obtained from a media formulation database 580 together with predefined criteria (e.g. upper and lower limits) on total impurity levels in the respective cell culture medium (see action 530, "matching trait target specification rages"). Subsequently, only impurity levels in these sensitive raw materials may be considered for optimizing the initial ranges on the impurity levels obtained from the raw material database, whereas the total impurity trait content calculation of the medium considers impurity content of all raw materials of the formulation (see action 540). This may involve an optimization algorithm to ensure that the probability $p_{viol}$ may be equal to or below a predefined threshold probability. Based on these optimized raw material limits for particular impurity levels (see action 550), the raw material database may be updated for ensuring a more robust raw material sourcing (see action 560).

The actions 510 to 560 as illustrated by flow chart 500 may for example be understood as an optimization algorithm which enables to go back to raw material level by using inputs for media level. In general, the optimization algorithm may use the targeted accepted level of an impurity in a cell culture medium and data for different batches of raw materials in the medium to find optimized values per raw material per impurity to minimize the violation of specific, predefined criteria or boundaries in the cell culture medium for respective impurity levels. For example, the media specification, media recipe and limited measurement of raw material batches are inputted and as an output the optimization algorithm provides the specification (i.e. allowable ranges) on the level of raw materials. In particular, an exemplary overall target may be to optimize raw material specification limits to ensure that a cell culture medium complies with predetermined specifications (e.g. with a probability equal to or higher than a required threshold probability).

For example, the actions 510 to 560 as illustrated by flow chart 500 may for example be understood as part of a self-learning and continuously improving system. The procedure may be repeated regularly, for example when adding new materials, new raw material batches or new media formulations to the step to derive more refined and robust ranges.

Fig. 6 shows a flow chart 600 illustrating another exemplary embodiment of the method according to the present disclosure. For example, the actions of flow chart 600 may be performed as part of determining allowable ranges for respective impurity levels in raw materials as described for action 410 in flow chart 400 shown in Fig. 4 and for an optimization of raw material limits as described for flow chart 500 shown in Fig. 5. In particular, the actions of flowchart 600 may be understood as subpart of the method shown in flow chart 500, wherein the actions according to flow chart 600 may be performed after selecting limits only for sensitive raw materials in action 540 and before optimizing raw material limits in action 550 according to flow chart 500. Without limiting the scope of the present disclosure, it may be assumed that the actions of flow chart 600 may be performed by an apparatus as described with reference to Fig. 12.

Considering for example an initial range of respective impurity levels ("RM trait values from specified range") in a raw material, Gaussian sampling or other sampling forms may be used to compute resulting media specification values (see actions 610 and 620). For example, a number of values (e.g. $n_{stat} \geq 10^3$ for reliable statistics) according to a Gaussian distribution within the initial range may be selected and based on these values, a probability $p_{viol}$ (e.g. an out-of-specification, OOS, probability for the target medium) may be determined that a total impurity level in the cell culture medium may fail the at least one predefined criterion (see action 630). For example, the at least one predefined criterion may be failed if a deviation of the determined total impurity level from a reference total impurity level in the cell culture medium (e.g. expressed by an objective function $f_{obj,k}^{Trait}$) is larger than an upper limit An example for a loop count for the out-of-specification results is given by the following pseudo-code:

$$\text{for } k = 1 \text{ to } n_{\text{Traits}}$$

$$\text{OOS\_count} (k) = 0$$

$$\text{for } k = 1 \text{ to } n_{\text{Stats}}$$

$$\text{Calculate } f_{\text{obj},k}^{\text{Trait}}$$

$$\text{if } f_{\text{obj},k}^{\text{Trait}} > 0$$

$$\text{OOS\_count} (k) += 1$$

$$\text{endif}$$

$$\text{end}$$

In this example, the violation probability may be calculated according to $p_{\text{viol,k}} = \dfrac{\text{OOS\_count}(k)}{n_{\text{stats}}}$. Subsequently, it may be checked whether the violation probability is below a predefined probability threshold (i.e. $p_{\text{viol,k}} \leq p_{\text{threshold}}$). For example, $p_{\text{threshold}} = 0.05$ would mean that at least 95% of produced media batches would be expected to meet the required specifications. Subsequently, given that the violation probability is below a predefined probability threshold, an allowable range for the respective impurity level in the raw material is for example determined by calculating and minimizing an objective function including the violation probability weighted by a weighting factor $K$ (e.g. $10^5$) and a penalty term that ensures that the optimization algorithm computes impurity levels as wide as possible for the allowable range of the optimized raw materials (e.g. instead of deriving a minimum allowable range for minimizing the deviation of a total impurity level from a reference total impurity level, see action 640). The corresponding raw material impurity level limits from the allowable range may then be used to update the raw material database as described above with reference to Fig. 5. For example, the actions illustrated in Fig. 6 may be referred to CMA-ES loop, which may be repeated for several generations $n_{\text{gen}}$ and further for several restarts $n_{\text{restart}}$ with various initial ranges to address potential local minima.

The exemplary loop count for the out-of-specification results given by the pseudo-code above may be understood as inner loop, which may be part of an optimization algorithm that may use batch raw material trait data on impurity levels for calculating mean value and standard deviation of the respective trait per raw material, for example by averaging over the different batches and correspondingly the media trait value. The inner loop may then be performed to calculate (e.g. compute) the percentage of out of specification media, which may correspond to $p_{\text{viol,k}}$ as described above by defining a function as a metric to count out of specification events.

Further regarding an inner loop workflow, the objective function may for example also be formulated in a way that it may be tracked if a given media trait (i.e. a particular total impurity) is below or above a defined media trait specification range (e.g. according to a predefined criterion of a cell culture medium). In such an exemplary evolutionary strategy set up, the optimization algorithm may try to minimize the objective function value (fitness value), which is directly dependent on a violation probability by optimizing/adapting the mean $\mu$ and standard deviation $\sigma$ of the specification ranges of a given trait for a set of raw materials (i.e. the corresponding impurity levels in a plurality of raw materials). To give a non-limiting example, a corresponding objective function may be:

$$K \cdot f_{\text{obj},k}^{\text{Trait}} + f_{\text{obj}}^{\text{penalty}} = K \cdot \sum_{k=1}^{n_{\text{Traits}}} f_{\text{obj},k}^{\text{Trait}} + f_{\text{obj}}^{\text{penalty}} = \min!$$

$$= \sum_{k}^{n_{\text{Traits,opt}}} \sum_{l=1}^{n_{\text{Raw Materials,opt}}} \left| \frac{\max(\sigma_{l,k}^{n=0}, \sigma_{l,k}^{n}) - \sigma_{l,k}^{n}}{\max(\sigma_{l,k}^{n=0}, \sigma_{l,k}^{n})} \right| + K \cdot \max(p_{\text{viol,k}} - p_{\text{threshold}}, 0).$$

In particular, this exemplary objective function depends on $p_{\text{viol,k}}$, which the optimization algorithm tries to minimize. The objective function may sum the scaled contributions of all media traits (i.e. all impurities) to be optimized (e.g. bioburden and manganese content) and for each trait over all raw materials contributing to the total impurity level in the cell culture medium. The penalty term $f_{\text{obj}}^{\text{penalty}}$ containing the sigma expressions may favour keeping raw material specification ranges as broad as possible - which may for example be desirable from a commercial standpoint - while ensuring adherence to the upper limit given on acceptable violation probabilities (i.e. the maximum percentage of expected out of specification batches in media production).

The violation of media specification may occur in different cases as in a single direction (higher than upper limit/lower than lower limit) or in both directions. The optimization algorithm may provide different treatments based on different cases. In the former case, optimization of $\mu$ (adaptation of $\sigma$) and in the latter case optimization of only $\sigma$ may be targeted. Such a switch strategy may support the convergence of the algorithm if one of the scenarios alone is able to sufficiently decrease the violation probability. Once the optimization is successful, the optimized specification ranges are calculated as follows per raw material.

$$\left[ \mu_{i,n}^{j,\text{pot}} - \alpha_{i,j} \cdot \sigma_{i,n}^{j,\text{opt}}, \mu_{i,n}^{j,\text{opt}} + \alpha_{i,j} \cdot \sigma_{i,n}^{j,\text{opt}} \right]$$

In particular, $\alpha_{i,j}$ may be considered to be equal to 3 without loss of generality. For example, a range of $3\sigma$ for a normally distributed random trait variation in a raw material would result in 99% of raw material batches sourced fulfilling this specification.

Fig. 7a, 7b and to 7c show another exemplary embodiment of the method according to the present disclosure. For example, Figs. 7a to 7c may refer to an exemplary proof of concept of the method according to the present disclosure as further described with reference to Fig. 4, 5 and 6.

A commonly used cell culture media formulation, namely a slightly altered RMPI open source medium given in Fig. 7a may be used as an input to illustrate the application of the algorithm described with reference to Fig. 4, 5 and 6. The exemplary medium has 38 compounds (i.e. raw materials), wherein two traits (i.e. two impurities) Manganese and Nickel of respective trait values (i.e. impurity levels) are selected. Fig. 7b shows the status of the convergence (i.e. fitness values) for different restart iterations of the evolutionary strategy during optimization. Therein, each restart may be seen as an independent set of optimization simulations. As shown in Fig. 7b, in the first 15 restarts, the algorithm was started with optimizing $\mu$ and adapting $\sigma$ correspondingly. In this case, significant improvement of the violation probability may be achieved for some settings, but not sufficiently to reach the acceptance criterion (e.g. below 10% violation probability), since some raw materials still led to exceeding and underachieving the target specification range. Therefore, further optimizations may be conducted, by switching the strategy where only the standard deviation $\sigma$ of the raw material traits was started. The best convergence is happening at restart 18 where $\sigma$ has been optimized. A summary of the algorithm application according to this example is provided in Fig. 7c for some sensitive compounds. In this example, the algorithm tries to reach a global minimum by modifying $\mu$ and $\sigma$ for different raw materials and most significantly as it is shown in Fig. 7c, decreases $\mu$ and $\sigma$ for the two sensitive compounds for both cases to be able to decrease violation probability.

Fig. 8 shows a flow chart 800 illustrating another exemplary embodiment of the method according to the present disclosure. For example, action 810 may be performed after action 330 or after action 340 as described with reference to Fig. 3. Without limiting the scope of the present disclosure, it may be assumed that action 810 of flow chart 800 may be performed by an apparatus as described with reference to Fig. 12.

Action 810 is determining a respective preferred batch of at least one raw material of the plurality of raw materials, wherein, at least partially based on the impurity level of the at least one impurity in the preferred batch of the at least one raw material, a deviation of the total impurity level and/or the at least one further total impurity level in the cell culture medium from a respective reference total impurity level in the cell culture medium is reduced, in particular by using an optimization algorithm for minimizing the deviation of the total impurity level and/or the at least one further total impurity level from the respective reference total impurity level.

For example, minimizing the deviation from the respective reference total impurity level may be understood to mean that the deviation is minimized to be within a range around the total reference impurity level.

Considering the example described with reference to Fig. 2a, batch A2 may be determined as preferred batch of raw material A, wherein the deviation of the total impurity level of Mn in the cell culture medium from the respective reference total impurity level of Mn is reduced (e.g. reduced compared to when batch A1 would be used to determine the total impurity level of Mn in the cell culture medium). For example, determining a preferred batch of a raw material may be understood to mean that a preferred batch is determined based on which the deviation of the total impurity level from the respective reference total impurity is minimized (e.g. at least locally minimized). Regarding a sufficiently large plurality of raw materials, determining the preferred batch of a raw material may require using an optimization algorithm as further described in the following.

As for example described with reference to Figs. 2a and 2b, the following objective function may be used to express a deviation between a determined total impurity level (e.g. referred to as simulated trait) and a total reference impurity level (e.g. referred to as reference trait).

$$f_{\text{obj}}^{\text{Media A}} = \sum_{i=1}^{n} w_i \left. \left( \left( \text{Trait}_i^{\text{Media A,Ref}} - \text{Trait}_i^{\text{Media A,Sim}} \right)^2 \right) \middle/ \left( \text{Trait}_i^{\text{Media A,Ref}} \right)^2 \right.,$$

wherein the value of $\text{Trait}_i^{\text{Media A,Sim}}$ may be given by

$$\text{Trait}_i^{\text{Media A,Sim}} = \text{Trait}_{i,j,k}^{\text{Media A}} = \sum_{j=1}^{n} {}^{\text{RM}} w_{ji} \cdot \text{Trait}^{\text{RM}_j} \cdot F(k).$$

For including or not including a batch of a particular raw material in the cell culture medium to be optimized, $k$ in $F(k)$ may be 0 (i.e. the batch is not included) or 1 (i.e. the batch is included). For example, two raw materials with three batches each may be represented by a set [A1 A2 A3 B1 B2 B3] of a cell culture medium, wherein the set [1 0 0 0 1 0] may indicate that batch A1 of raw material A and batch B2 of raw material B is included in the cell culture medium. In one example, a preferred batch of raw material A may be determined by calculating $f_{\text{obj}}^{\text{Media A}}$ for every possible combination [A1 A2 A3 B1 B2 B3] of batches within the cell culture medium (which are e.g. nine combinations for two raw materials with three batches for each raw material). The preferred batch of a particular raw material may then be determined from the combination of batches that leads to the minimum value of $f_{\text{obj}}^{\text{Media A}}$. To improve the efficiency of such an approach if a larger number of raw materials (e.g. 50 raw materials) in a cell culture medium with respective batches is assessed, not each raw material of the plurality of raw materials may be considered (see $\text{Trait}^{\text{RM}}_j$ for $n$ raw materials in the cell culture medium), but only those raw materials whose contribution to the corresponding total impurity level is larger than a predefined threshold.

Another approach of improving efficiency when determining a preferred batch may be to use a global optimizer instead of determining $f_{\text{obj}}^{\text{Media A}}$ for every possible combination of batches. For example, such a global optimizer may follow a customized covariance matrix adaptation evolution strategy (CMA-ES, e.g. developed from an evolutionary strategy known in literature) as further described with reference to Fig. 8.

Fig. 9 shows a scheme illustrating another exemplary embodiment of the method according to the present disclosure. For example, the approach according to Fig. 9 may be part of determining a preferred batch as described for action 810 in flow chart 800 shown in Fig. 8.

As described with reference to Fig. 8, a customized CMA-ES optimization algorithm may be used to minimize $f_{\text{obj}}^{\text{Media A}}$ for determining a preferred batch of at least one raw material. Since evolutionary optimization algorithms like CMA-ES typically solve continuous optimization problems, a conversion function 920 as part of a customized CMA-ES 900 may be used for solving a discrete problem (e.g. the selection of a preferred batch).

An exemplary application of a conversion function 920 may be understood with reference to Fig. 9. In each iteration of the CMA-ES optimization algorithm 900, the temporary result of the optimization algorithm may be given by a variable x in form of a tensor 910. The conversion function 920 may then map x to corresponding values of $k$, wherein $k$ indicates the selection of a particular batch. In particular, x is a continuous variable representing the choice of batches in the interval [1, $n_{\text{batches}}$]. The conversion function 920 as mapping function then ensures that the continues x values in the interval are mapped to the respective integer batch numbers 930 with equal probability. The term *"x before"* indicates the variable x as generated by a previous iteration of the algorithm.

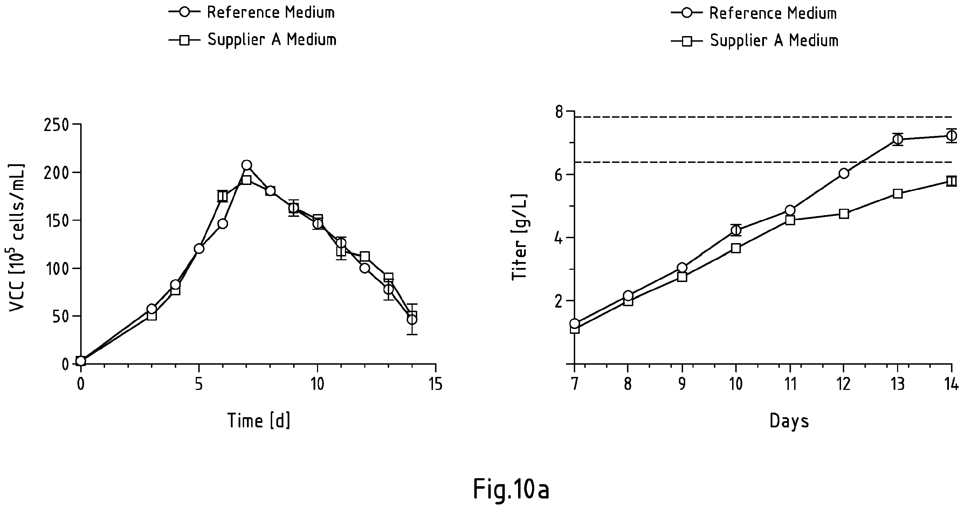

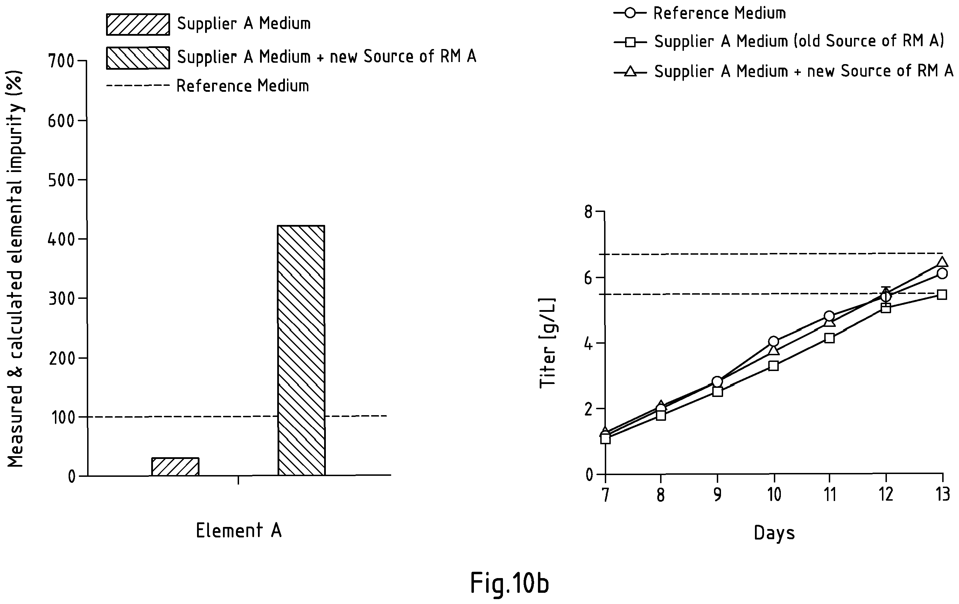

Figs. 10a and 10b show exemplary experimental data according to the present disclosure.

As a non-limiting example for a cell culture medium to be optimized, a fed batch culture medium used to culture Chinese hamster ovary (CHO) cells is inspected. CHO cells, genetically modified to express a product, in this case an antibody, were provided. A batch of cell culture medium was inoculated with the CHO cells at $3 \times 10^5$ cells per mL of cell culture medium and incubated in a cell culture incubator at usual cell culture conditions (e.g. 36.8 °C $\pm$ 0.2 °C, 7.5 % $\pm$ 0.5 % $CO_2$, 110 rpm $\pm$ 5 rpm) in a fed-batch process for a period of 14 days. Starting from day 3 of culture, the viable cell concentration (VCC) was determined daily using a cell counter such as a Vi-CELL XR Cell Viability Analyzer (Beckman Coulter). Additionally, starting from day 7 of culture, the product titer (i.e.: the amount of produced antibody per volume of cell culture medium) was determined by means of bilayer interferometry, e.g. using an Octet device (Sartorius Molecular Devices).

Fig. 10a shows the performance of a reference medium in comparison to a medium having the same general composition but consisting of differently sourced raw materials ("supplier A" as denoted in Fig. 10a). Therein, Fig. 10a on the left shows that both the reference medium and the supplier A medium provided comparable viable cell concentrations (VCC) of up to 200 x $10^5$ cells per mL of cell culture medium on day 7 of culture, which descended as the cell culture period extended beyond day 7 of culture. However, Fig. 10a on the right shows that the resulting product titer in supplier A cell culture medium after 14 days of culture was approx. 20 % lower than the product titer in the reference medium.

In order to improve the supplier A cell culture medium, the impurity level of at least one impurity in each raw material of supplier A cell culture medium was determined. Moreover, the formulation of supplier A cell culture medium was obtained. From this data, the total impurity level in the supplier A cell culture medium of the at least one impurity was determined, and it was further determined whether the total impurity level meets a predefined criterion. It was shown that the functional impurity "element A" in the Supplier A cell culture medium was present at a concentration below the total impurity level of element A in the reference medium, wherein the total impurity level of element A in the reference medium is outlined as horizontal dashed line in Fig. 10b on the left.

Further, it was determined by carrying out the method according to the present disclosure that raw material A ("RM A") had the largest contribution to the deviation of the total impurity level of element A in the supplier A cell culture medium from the total impurity level of element A in the reference medium. Subsequently, by use of the method according to the present disclosure, a preferred batch of RM A was determined. This preferred batch had an impurity profile leading to a total impurity level of element A higher than the total impurity level of element A in the reference medium. In this example, the reduced deviation is given by exceeding the total impurity level of element A in the reference medium. Fig. 10b on the left shows that the supplier A cell culture medium contains element A in a concentration below the total impurity level of element A in the reference medium, and that the optimized supplier A cell culture medium comprising a preferred batch of RM A (referenced in Fig. 10b as "Supplier A + new source of RM A") contains element A in a concentration larger than the total impurity level of element A in the reference medium (i.e. thereby meeting the predefined criterion).

In a subsequent experiment, which was carried out under similar conditions as described above with reference Fig 10a, the product titer was determined every 24 hours starting from day 7. Fig. 10b on the right shows the resulting product titers. Therein, the results showed that the supplier A cell culture medium (referred to as "Supplier A Medium (old Source of RM A)" in Fig. 10b on the right) had the lowest titer after 13 days of culture, while the cell culture medium optimized by the method according to the present disclosure (referred to as "Supplier A + new Source of RM A" in Fig. 10b on the right) produced a higher product titer which was approximately equal to the one observed for the reference medium.

These experimental data show that the method according to the present disclosure is capable of improving the performance of cell culture media, for example by increasing the productivity of cells cultured in the optimized cell culture media, as evidenced by increased product titers compared to a cell culture medium to which the method was not applied.

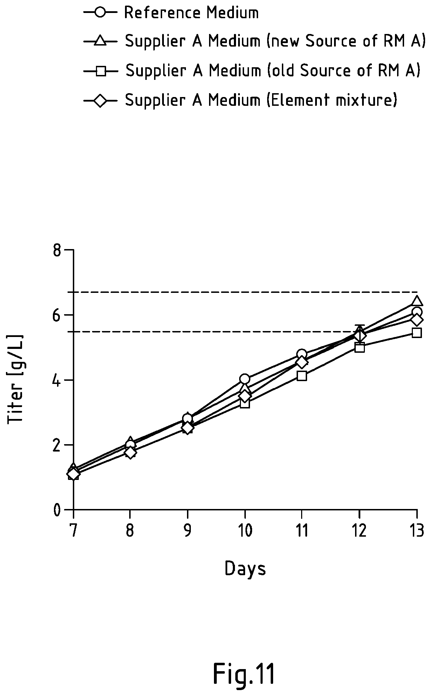

Fig. 11 shows further exemplary experimental data according to the present disclosure.

As a non-limiting example, the method according to the present disclosure as described with reference to Figs. 10a and 10b was compared to a method of the prior art for improving cell culture media. It is a known procedure to supplement cell culture media with trace elements for improving the performance of a cell culture. To this end, in a comparative experiment, the supplier A cell culture medium was supplemented with an element mixture, including element A and other elements. The selected elements were suggested by the method according to the present disclosure based on the contribution of RM A to the impurity level of those elements (see Fig. 11: "Supplier A Medium (Element mixture)").

Fig. 11 shows the resulting product titers after up to 13 days of culture. Especially, the cell culture medium with new source of RM A resulted in a higher product titer than the reference medium (see Fig. 11: "Supplier A Medium (new Source of RM A)"). Meanwhile, the medium generated by supplementing Supplier A cell culture medium with element A and other elements ("Supplier A Medium (Element mixture)") showed a comparable product titer after 13 days of culture to the reference medium. These findings indicate that the increase in the product titer can be achieved by either replacing RM A or supplementing the element mixture, suggested by the method according to the present disclosure.

The example illustrated in Fig. 11 shows that by using the method according to the present disclosure, the quality of cell culture can be optimized, as evidenced by the high cell culture product titer achieved with the cell culture medium optimized by the method of the present disclosure.

Fig. 12 shows a schematic illustration of an exemplary embodiment of an apparatus according to the present disclosure.

The apparatus 1200 comprises, by way of example, a processor 1201 and, connected to the processor 1201, a first memory as a program and data memory 1202, a second memory as a main memory 1203, a communication interface 1204 and a user interface 1205.

A processor is intended to be understood to mean, by way of example, a microprocessor, a microcontrol unit, a microcontroller, a digital signal processor (DSP), an application specific integrated circuit (ASIC) or a field programmable gate array (FPGA). It goes without saying that the apparatus 1200 can also comprise multiple processors 1201.

Processor 1201 executes programming instructions stored in program memory 1202 and stores, by way of example, intermediate results or the like in main memory 1203. The program memory 1202 contains, by way of example, program instructions of a computer program (e.g. a computer program according to the present disclosure) that cause the processor 1201 to perform and/or control a method disclosed according to the present disclosure when the processor 1201 executes these program instructions stored in program memory 1202. Moreover, program memory 1202 may store, by way of example, one or more databases (e.g. a raw material database and/or a media formulation database) or representations or one or more databases.

Program memory 1202 further contains, by way of example, the operating system of apparatus 1200, which is loaded at least in part into main memory 1203 when the apparatus 1200 is started, and is executed by the processor 1201. In particular, when apparatus 1200 is started, at least a part of the core of the operating system is loaded into the main memory 1203 and executed by processor 1201.

An example of an operating system is a Windows, UNIX, Linux, Android, Apple iOS and/or MAC OS operating system. The operating system in particular allows the use of the control apparatus for information and/or data processing. By way of example, it manages resources such as a main memory and a program memory, uses programming interfaces, inter alia, to provide other computer programs with fundamental functions and controls the execution of computer programs.

A program memory is, by way of example, a non volatile memory such as a Flash memory, a magnetic memory, an EEPROM (Electrically Erasable Programmable Read Only Memory) and/or an optical memory. A main memory is, for example, a volatile or non volatile memory, in particular a random access memory (RAM) such as a static RAM (SRAM), a dynamic RAM (DRAM), a ferroelectric RAM (FeRAM) and/or a magnetic RAM (MRAM).

Main memory 1203 and program memory 1202 may also be designed as one memory. Alternatively, main memory 1203 and/or program memory 1202 may each be formed by multiple memories. Further, main memory 1203 and/or program memory 1202 may also be part of the processor 1201.

Processor 1201 controls the communication interface 1204, which is designed as a wireless and/or wired communication interface, for example. A wireless and/or wired communication interface can, by way of example, receive information (via a wireless and/or wired communication path) and forward it to the processor 1201 and/or can receive information from the processor 1201 and send it (via a wireless and/or wired communication path).

An example of a wireless communication interface is a wireless network adapter. For example, a wireless communication interface comprises, besides an antenna, at least one transmitter circuit and one receiver circuit or a transceiver circuit. Examples of a wireless communication interface are a GSM, UMTS, LTE and/or 5G interface and/or a WLAN and/or Bluetooth interface. As disclosed above, the GSM, UMTS, LTE and 5G specifications are maintained and developed by the 3rd Generation Partnership Project (3GPP) and are currently available on the Internet atwww.3gpp.com. WLAN is specified in the standards of the IEEE 802.11 family, for example. The Bluetooth specifications are currently available on the Internet at www.bluetooth.org.

An example of a wired communication interface is a wired network adapter. For example, a wired communication interface comprises at least one transmitter circuit and one receiver circuit or a transceiver circuit An example of a wired communication interface is an Ethernet interface. Ethernet is specified in the standards of the IEEE 802.3 family, inter alia.

For example, the communication interface 1204 is configured for receiving information and/or for sending information (e.g. from another apparatus).

Further, processor 1201 controls the user interface 1205, which is configured for capturing information in the form of a user input (e.g. as a keyboard or mouse input and/or as a voice input and/or as a gesture) and/or for outputting information in the form of a, by way of example, audible and/or visual user output (e.g. as a voice output and/or as a text output). A user interface is a keyboard, a mouse, a camera, a screen, a touch sensitive screen, a loudspeaker and/or a microphone, for example.

The components 1201 to 1205 of apparatus 1200 are communicatively and/or operatively connected to one another via one or more bus systems (e.g. one or more serial and/or parallel bus connections), for example.

In further examples, apparatus 1200 may comprise further components besides the components 1201 to 1205.

To give some non-limiting examples, apparatus 1200 could be understood as a server or a terminal device (e.g. a stationary device such as e.g. personal computer or a mobile device such as e.g. a telephone, a smartphone, a tablet, a laptop computer or similar). In other examples, apparatus 1200 could equally be a component, like a chip, circuitry on a chip or a plug-in board, for any electronic device.

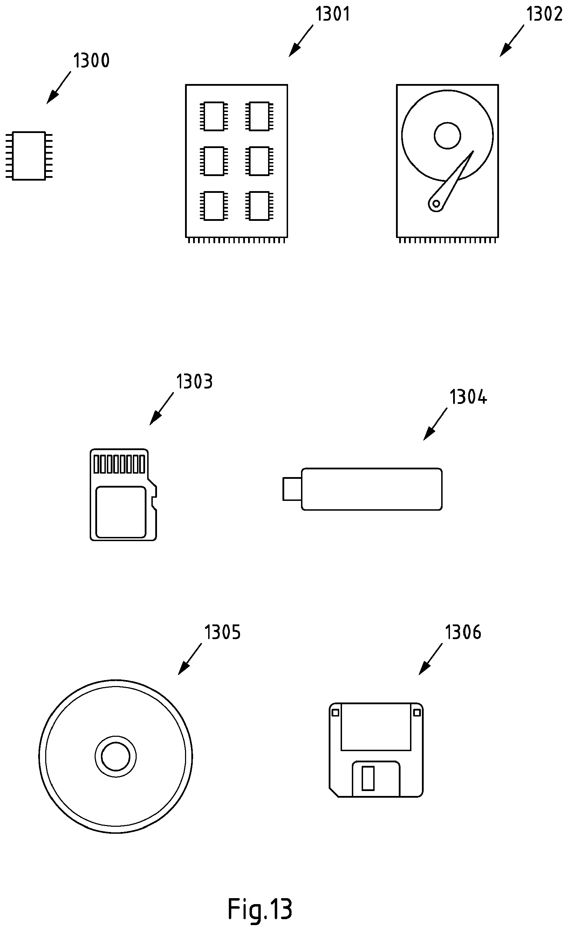

Fig. 13 shows exemplary embodiments of storage media. The storage medium may, by way of example, be a magnetic, electrical, optical and/or other kind of storage medium. The storage medium may, by way of example, be part of a processor (e.g. the processor 1201 of apparatus 1200 from Fig. 12), for example a (non volatile or volatile) program memory of the processor or a part thereof (e.g. program memory 1202). Exemplary embodiments of a storage medium are a flash memory 1300, an SSD hard disc 1301, a magnetic hard disc 1302, a memory card 1303, a memory stick 1304 (e.g. a USB stick), a CD ROM or DVD 1305 of a floppy disc 1306.

The following embodiments are disclosed as exemplary embodiments of the present disclosure:

Exemplary embodiment 1:

A computer-implemented method comprising:

- obtaining respective impurity levels of at least one impurity in each raw material of plurality of raw materials;
- obtaining a formulation of a cell culture medium, wherein the formulation indicates a ratio of the plurality of raw materials in the cell culture medium; and
- determining, at least partially based on the formulation and on the respective impurity levels, a total impurity level of the at least one impurity in the cell culture medium.

Exemplary embodiment 2:

The method according to embodiment 1, the method further comprising:

- determining, at least partially based on the formulation and on respective impurity levels of at least one further impurity in each raw material of the plurality of raw materials, at least one further total impurity level of the at least one further impurity in the cell culture medium.

Exemplary embodiment 3:

The method according to embodiment 1 or embodiment 2, the method further comprising:

- causing measuring at least one impurity level of the respective impurity levels in each raw material of the plurality of raw materials; and/or
- outputting information indicating the total impurity level of the at least one impurity in the cell culture medium and/or the at least one further total impurity level of the at least one further impurity in the cell culture medium.

Exemplary embodiment 4:

The method according to any of the embodiments 1 to 3, wherein the respective impurity levels in each raw material refer to a respective batch of the respective raw material.

Exemplary embodiment 5:

The method according to any of the embodiments 1 to 4, the method further comprising:

- determining, at least partially based on the total impurity level in the cell culture medium, the formulation and the respective impurity levels, a respective contribution of one or more raw materials of the plurality of raw materials to the total impurity level and/or to the at least one further total impurity level in the cell culture medium.

Exemplary embodiment 6:

The method according to embodiment 5, the method further comprising:

- outputting information indicating one or more raw materials whose contribution to the total impurity level and/or to the at least one further total impurity level is larger than a predefined threshold.

Exemplary embodiment 7:

The method according to any of the embodiments 1 to 6, the method further comprising:

- determining whether the total impurity level and/ or the at least one further total impurity level meet at least one predefined criterion.

Exemplary embodiment 8:

The method according to embodiment 7, wherein determining whether the total impurity level and/or the at least one further total impurity meet at least one predefined criterion further comprises at least one of the following:

- determining whether the total impurity level and/or the at least one further total impurity level are below a predefined upper limit in the cell culture medium; and/or
- determining whether the total impurity level and/or the at least one further total impurity level are above a predefined lower limit in the cell culture medium.

Exemplary embodiment 9:
The method according to any of the embodiments 7 and 8, the method further comprising:

- determining, if it is determined that the total impurity level of a functional impurity in the cell culture medium does not meet the at least one predefined criterion, that the functional impurity is to be added to the cell culture medium; and, in particular,
- outputting information indicating that the functional impurity is to be added to the cell culture medium.

Exemplary embodiment 10:
The method according to any of the embodiments 7 to 9, wherein determining whether the total impurity level and/or the at least one further total impurity level meet at least one predefined criterion is at least partially based on an upstream process model and/or on a downstream process model of the cell culture medium.

Exemplary embodiment 11:
The method according to any of the embodiments 5 to 10, the method further comprising:

- if it is determined that the total impurity level and/or the one further total impurity level meet the at least one predefined criterion, outputting information indicating the plurality of raw materials and the formulation as suitable for manufacturing the cell culture medium.

Exemplary embodiment 12:
The method according to any of the embodiments 1 to 11, the method further comprising:

- determining, at least partially based on the formulation, on the respective impurity levels in each raw material of the plurality of raw materials and on at least one predefined criterion, a respective allowable range of the impurity level of the at least one impurity in at least one raw material of the plurality of raw materials.

Exemplary embodiment 13:
The method according to embodiment 12, wherein determining the respective allowable range of the impurity level of the at least one impurity in at least one raw material of the plurality of raw materials comprises:

- calculating, at least partially based on an initial range of the impurity level of the at least one impurity in the at least one raw material, a probability that the total impurity level of the at least one impurity in the cell culture medium fails the at least one predefined criterion; and
- determining, in particular by using an optimization algorithm, the respective allowable range of the impurity level based on the calculated probability and the initial range of the impurity level.

Exemplary embodiment 14:
The method according to embodiment 12 or embodiment 13, wherein the method further comprises:

- outputting information indicating the respective allowable range of the impurity level in the at least one raw material.

Exemplary embodiment 15:
The method according to any of the embodiments 1 to 14, the method further comprising:

- determining a deviation of the total impurity level and/or the at least one further total impurity level in the cell culture medium from a respective reference total impurity level in the cell culture medium.

Exemplary embodiment 16:
The method according to embodiment 15, the method further comprising:

- determining, at least partially based on the total impurity level and/or the at least one further total impurity in the cell culture medium, the formulation and the respective impurity levels in each raw material, a respective contribution of one or more raw materials of the plurality of raw materials to the determined deviation; and
- determining one or more raw materials whose contribution to the determined deviation is larger than a predefined threshold.

Exemplary embodiment 17:
The method according to any of the embodiments 1 to 16, the method further comprising:

- determining a respective preferred batch of at least one raw material of the plurality of raw materials, wherein, at least partially based on the impurity level of the at least one impurity in the preferred batch of the at least one raw material, a deviation of the total impurity level and/or the at least one further total impurity level in the cell culture medium from a respective reference total impurity level in the cell culture medium is reduced, in particular by using an optimization algorithm for minimizing the deviation of the total impurity level and/or the at least one further total impurity level from the respective reference total impurity level.

Exemplary embodiment 18:
The method according to embodiment 17, wherein a respective preferred batch of the one or more raw materials whose contribution to the determined deviation is larger than a predefined threshold is determined.

Exemplary embodiment 19:
The method according to any of the embodiments 1 to 18, the method further comprising:

- determining a respective equivalent raw material of at least one raw material of the plurality of raw materials, wherein, at least partially based on the impurity level of the at least one impurity in the respective equivalent raw material, a deviation of the total impurity level and/or the at least one further total impurity level in the cell culture medium from a respective reference total impurity level in the cell culture medium is reduced, in particular by using an optimization algorithm for minimizing the deviation of the total impurity level and/or the at least one further total impurity level from the respective reference total impurity level.

Exemplary embodiment 20:
The method according to any of the embodiments 1 to 19, wherein a plurality of formulations of respective cell culture media is obtained, wherein each formulation of the plurality of formulations indicates a ratio of a plurality of raw materials in the respective cell culture medium; and wherein the method further comprises at least one of the following:

- determining the respective allowable range of the impurity level of the at least one impurity based on the plurality of formulations; and/or
- determining a respective preferred batch of the at least one raw material based on the plurality of formulations.

Exemplary embodiment 21:
The method according to any of the embodiments 1 to 20, wherein the at least one impurity and/or the at least one further impurity is one of the following:

- a toxic impurity; or
- a functional impurity; or
- an organic compound; or
- an inorganic compound; or
- bioburden; or
- endotoxin.

Exemplary embodiment 22:
The method according to any of the embodiments 1 to 21, wherein the cell culture medium is of liquid form and wherein the formulation of the cell culture medium further indicates a hydration procedure for the plurality of raw materials.

Exemplary embodiment 23:
A computer program comprising instructions which, when the computer program is executed by a computer, cause the computer to carry out the method of any of the embodiments 1 to 22.

Exemplary embodiment 24:
An apparatus comprising means for carrying out the method of any of the embodiments 1 to 22.

It will be understood that the embodiments disclosed herein are only exemplary, and that any feature presented for a particular exemplary embodiment may be used with the present disclosure on its own or in combination with any feature presented for the same or another particular exemplary embodiment and/or in combination with any other feature not mentioned. It will further be understood that any feature presented for an example embodiment in a particular category may also be used in a corresponding manner in an example embodiment of any other category.

In the present disclosure, the wording "A, or B, or C, or a combination thereof' or "at least one of A, B and/or C" may be understood to be not exhaustive and to include at least the following: (i) A, or (ii) B, or (iii) C, or (iv) A and B, or (v) A and C, or (vi) B and C, or (vii) A and B and C.

**Claims**

1. A computer-implemented method (300) comprising:

   - obtaining (310) respective impurity levels of at least one impurity in each raw material of a plurality of raw materials (110;120;130);
   - obtaining (320) a formulation (140) of a cell culture medium, wherein the formulation (140) indicates a ratio of the plurality of raw materials (110;120;130) in the cell culture medium; and
   - determining (330), at least partially based on the formulation (140) and on the respective impurity levels, a total impurity level of the at least one impurity in the cell culture medium.

2. The method (300) according to claim 1, the method further comprising:

   - determining, at least partially based on the formulation (140) and on respective impurity levels of at least one further impurity in each raw material of the plurality of raw materials (110;120;130), at least one further total impurity level of the at least one further impurity in the cell culture medium.

3. The method (300) according to claim 1 or claim 2, the method further comprising:

   - causing measuring at least one impurity level of the respective impurity levels in each raw material of the plurality of raw materials (110;120;130); and/or
   - outputting information indicating the total impurity level of the at least one impurity in the cell culture medium and/or the at least one further total impurity level of the at least one further impurity in the cell culture medium.

4. The method (300) according to any of the claims 1 to 3, the method further comprising:

   - determining, at least partially based on the total impurity level in the cell culture medium, the formulation (140) and the respective impurity levels, a respective contribution of one or more raw materials of the plurality of raw materials (110;120;130) to the total impurity level and/or to the at least one further total impurity level in the cell culture medium; and
   - outputting information indicating one or more raw materials whose contribution to the total impurity level and/or to the at least one further total impurity level is larger than a predefined threshold.

5. The method (300) according to any of the claims 1 to 4, the method further comprising:

   - determining (340) whether the total impurity level and/ or the at least one further total impurity level meet at least one predefined criterion.

6. The method (300) according to claim 5, wherein determining whether the total impurity level and/or the at least one further total impurity level meet at least one predefined criterion is at least partially based on an upstream process model (150) and/or on a downstream process model (160) of the cell culture medium.

7. The method (300;400) according to any of the claims 1 to 6, the method further comprising:

   - determining (410), at least partially based on the formulation (140), on the respective impurity levels in each raw material of the plurality of raw materials (110;120;130) and on at least one predefined criterion, a respective allowable range of the impurity level of the at least one impurity in at least one raw material of the plurality of raw materials (110;120;130).

8. The method (300;400) according to claim 7, wherein determining (410) the respective allowable range of the impurity level of the at least one impurity in at least one raw material of the plurality of raw materials (110;120;130) comprises:

- calculating, at least partially based on an initial range of the impurity level of the at least one impurity in the at least one raw material, a probability that the total impurity level of the at least one impurity in the cell culture medium fails the at least one predefined criterion; and
- determining, in particular by using an optimization algorithm, the respective allowable range of the impurity level based on the calculated probability and the initial range of the impurity level.

9. The method (300) according to any of the claims 1 to 8, the method further comprising:

- determining a deviation of the total impurity level and/or the at least one further total impurity level in the cell culture medium from a respective reference total impurity level in the cell culture medium;
- determining, at least partially based on the total impurity level and/or the at least one further total impurity in the cell culture medium, the formulation (140) and the respective impurity levels in each raw material, a respective contribution of one or more raw materials of the plurality of raw materials (110;120;130) to the determined deviation; and
- determining one or more raw materials whose contribution to the determined deviation is larger than a predefined threshold.

10. The method (300;800) according to any of the claims 1 to 9, the method further comprising:

- determining (810) a respective preferred batch of at least one raw material of the plurality of raw materials (110;120;130), wherein, at least partially based on the impurity level of the at least one impurity in the preferred batch of the at least one raw material, a deviation of the total impurity level and/or the at least one further total impurity level in the cell culture medium from a respective reference total impurity level in the cell culture medium is reduced, in particular by using an optimization algorithm (900) for minimizing the deviation of the total impurity level and/or the at least one further total impurity level from the respective reference total impurity level.

11. The method (300;800) according to claim 10, wherein a respective preferred batch of the one or more raw materials whose contribution to the determined deviation is larger than a predefined threshold is determined.

12. The method (300) according to any of the claims 1 to 11, wherein a plurality of formulations (140) of respective cell culture media is obtained, wherein each formulation (140) of the plurality of formulations indicates a ratio of a plurality of raw materials (110;120;130) in the respective cell culture medium, and wherein the method further comprises at least one of the following:

- determining a respective allowable range of the impurity level of the at least one impurity based on the plurality of formulations (140); and/or
- determining a respective preferred batch of the at least one raw material based on the plurality of formulations (140).

13. The method (300) according to any of the claims 1 to 12, wherein the at least one impurity and/or the at least one further impurity is one of the following:

- a toxic impurity; or
- a functional impurity; or
- an organic compound; or
- an inorganic compound; or
- bioburden; or
- endotoxin.

14. A computer program comprising instructions which, when the computer program is executed by a computer, cause the computer to carry out the method (300) according to any of the claims 1 to 13.

15. An apparatus (1200) comprising means (1201;1202;1203) for carrying out the method (300) according to any of the claims 1 to 13.

Fig.1

| Raw Material | Batch | Mn impurity level [ppm] |
|---|---|---|
| Raw Material A (80%) | A1 | 4 |
| | A2 | 1.5 |
| | Aref | 1.6 |
| Raw Material B (20%) | B1 | 0.25 |
| | B2 | 2 |
| | Bref | 0.2 |

Fig.2a

EP 4 474 471 A1

| Raw Material | Batch | Mn impurity level [ppm] | Cu impurity level [ppm] |
|---|---|---|---|
| Raw Material A (40%) | A1 | 2 | 2 |
| | A2 | 1.5 | 2 |
| | Aref | 1.6 | 1 |
| Raw Material B (30%) | B1 | 0.25 | 0.25 |
| | B2 | 2 | 2 |
| | Bref | 0.2 | 0.3 |
| Raw Material C (30%) | C1 | 1.5 | 3 |
| | C2 | 1.2 | 2 |
| | C3 | 1 | 1 |
| | Cref | 0.6 | 0.6 |

Fig.2b

300

obtaining respective impurity levels of at least one impurity in each raw
material of a plurality of raw materials — 310

obtaining a formulation of a cell culture medium, wherein the formulation
indicates a ratio of the plurality of raw materials in the cell culture medium — 320

determining, at least partially based on the formulation and on the
respective impurity levels, a total impurity level of the at least one
impurity in the cell culture medium — 330

determining whether the total impurity level and/ or the at least one
further total impurity level meet at least one predefined criterion — 340

Fig.3

400

determining, at least partially based on the formulation, on the respective
impurity levels in each raw material of the plurality of raw materials and
on at least one predefined criterion, a respective allowable range of the
impurity level of the at least one impurity in at least one raw material
of the plurality of raw materials — 410

Fig.4

Fig.5

Raw Material (RM) Database — 570

Compute mean & std dev for raw material traits — 510

Identify sensitive raw materials affecting media specs — 520

Select only limits of sensitive RMs for Optimization — 540

Media Formulation Database — 580

Select Media Recipe + Matching Trait Target Specification Ranges — 530

Optimize RM limits ensuring Media is in spec with $P_{viol} \leq P_{threshold}$ — 550

Update RM spec limits In RM DB for more robust sourcing — 560

500

EP 4 474 471 A1

600

Gaussian sampling of
RM trait values from
specified range ⟋610

Compute resulting
Media Specification
Values ⟋620

Calculate avg.
Probability of OOS $P_{viol}$
for $f_{obj}^{Traits}$ and check
$P_{viol} \leq P_{threshold}$ ⟋630

Calculate $f_{obj}$
to minimize:
$K \cdot P_{viol} + $ Penalty Term ⟋640

Fig.6

| Raw material name | Content [g/l] |
|---|---:|
| Calcium Nitrate•4 $H_2O$ | 0.1 |
| Choline chloride | 0.003 |
| D(+)-Biotin | 0.0002 |
| D-Pantothenic Acid | 0.00025 |
| Folic acid | 0.001 |
| Glucose | 2 |
| Glycine | 0.01 |
| L-Arginine | 0.2 |
| L-Asparagine | 0.05 |
| L-Aspartic Acid | 0.02 |
| L-Cystine • 2HCl | 0.0652 |
| L-Glutamic Acid | 0.02 |
| L-Histidine | 0.015 |
| L-Isoleucine | 0.015 |
| L-Leucine | 0.05 |
| L-Lysine hydrochloride | 0.05 |
| L-Methionine | 0.04 |
| L-Phenylalanine | 0.015 |
| L-Proline | 0.015 |
| L-Serine | 0.02 |
| L-Threonine | 0.03 |
| L-Tryptophan | 0.02 |
| L-Valine | 0.02 |
| Myo-Inositol | 0.035 |
| Niacinamide | 0.001 |
| Phenol Red • Na | 0.0053 |
| Potassium Chloride | 0.4 |
| Pyridoxine Monohydrochloride | 0.001 |
| Riboflavin | 0.0002 |
| Sodium bicarbonate | 2 |
| Sodium Chloride | 6 |
| Sodium phosphate dibasic anhyd. | 0.8 |
| Thiamine HCL | 0.001 |
| Vitamin B12 | 0.000005 |
| Hydroxy-L-Proline | 0.02 |
| L-Tyrosine • 2Na • 2H2O | 0.02883 |
| Magnesium Sulfate (anhydrous) | 0.04884 |
| p-Aminobenzoic Acid | 0.001 |

Fig.7a

Fig.7b

| Case | acceptance criterion $P_{vio}$ | $P_{vio}$ | Mn sensitive compound 1 | Mn sensitive compound 2 | Ni sensitive compound 1 | Ni sensitive compound 2 |
|---|---|---|---|---|---|---|
| Restart =12 μ optimisation | 10% | Initial violation =70% Violation after optimization = 11% | Potassium chloride Initial μ=1.15 ppm Optimized μ=0.37 ppm | Glucose Initial μ=0.75 ppm Optimized μ= 0.2 ppm | Potassium chloride Initial μ=0.18 ppm Optimized μ= 0.02 ppm | Glucose Initial μ=0.07 ppm Optimized μ= 0.04 ppm |
| Restart =18 σ optimisation | 10% | Initial violation= 35% Violation after optimization = 7% | Potassium chloride Initial σ=0.63 ppm Optimized σ=0.19 ppm | Glucose Initial σ=0.35 ppm Optimized σ= 0.28 ppm | Potassium chloride Initial σ=0.17 ppm Optimized σ= 0.01 ppm | Glucose Initial σ= 0.042 ppm Optimized σ= 0.001 ppm |

Fig.7c

800

810

determining a respective preferred batch of at least one raw material of the plurality of raw materials, wherein, at least partially based on the impurity level of the at least one impurity in the preferred batch of the at least one raw material, a deviation of the total impurity level and/or the at least one further impurity level in the cell culture medium from a respective reference total impurity level in the cell culture medium is reduced, in particular by using an optimization algorithm for minimizing the deviation of the total impurity level and/or the at least one further total impurity level from the respective reference total impurity level

Fig.8

900

910 ⌐
```
x before: tf.Tensor(
[ [0.94586605   0.8328206 ]
  [0.93278265   0.833707   ]
  [0.93446803   0.82631457]
  [0.9225056    0.848811   ]
  [0.9424096    082525146 ] ], shape=(5, 2), dtype=float32)
```

Input in each interation
of CMA-ES

920 ⌐
Conversion Function

(Example for 2 RMs, each 3 batches)

Output in each interation
of CMA-ES

930 ⌐
```
array ( [ [0., 0., 1., 0., 0., 1.],
          [0., 0., 1., 0., 0., 1.],
          [0., 0., 1., 0., 0., 1.],
          [0., 0., 1., 0., 0., 1.],
          [0., 0., 1., 0., 0., 1.],], dtype=float32)
```

Fig.9

Fig.10a

EP 4 474 471 A1

Fig.10b

EP 4 474 471 A1

—○— Reference Medium
—△— Supplier A Medium (new Source of RM A)
—□— Supplier A Medium (old Source of RM A)
—◇— Supplier A Medium (Element mixture)

Fig.11

Fig.12

1300

1301

1302

1303

1304

1305

1306

Fig.13

**EP 4 474 471 A1**

Europäisches Patentamt
European Patent Office
Office européen des brevets

# EUROPEAN SEARCH REPORT

Application Number

EP 23 17 8373

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Woolstenhulme et al.: "An innovative approach to manufacturing custom CDM with precision accuracy", , 10 March 2023 (2023-03-10), XP093104664, Retrieved from the Internet: URL:https://www.cytivalifesciences.com/en/us/solutions/bioprocessing/knowledge-center/controlling-trace-impurities-in-chemically-defined-media [retrieved on 2023-11-17] * the whole document * ----- | 1-15 | INV. C12M1/36 B01F35/22 C12M1/00 C12N5/00 |
| A | BRUNNER MATTHIAS ET AL: "Towards robust cell culture processes – Unraveling the impact of media preparation by spectroscopic online monitoring", ENGINEERING IN LIFE SCIENCES, vol. 19, no. 10, 1 October 2019 (2019-10-01), pages 666-680, XP093102404, DE ISSN: 1618-0240, DOI: 10.1002/elsc.201900050 * the whole document * ----- | 1-15 | |
| A | RATHORE ANURAG S ET AL: "Role of raw materials in biopharmaceutical manufacturing: risk analysis and fingerprinting", CURRENT OPINION IN BIOTECHNOLOGY, LONDON, GB, vol. 53, 4 January 2018 (2018-01-04), pages 99-105, XP085497518, ISSN: 0958-1669, DOI: 10.1016/J.COPBIO.2017.12.022 * the whole document * ----- -/-- | 1-15 | TECHNICAL FIELDS SEARCHED (IPC) C12M C12N B01F |

The present search report has been drawn up for all claims

| Place of search | Date of completion of the search | Examiner |
|---|---|---|
| Berlin | 23 November 2023 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

48

Europäisches
Patentamt

European
Patent Office

Office européen
des brevets

**EUROPEAN SEARCH REPORT**

Application Number

EP 23 17 8373

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/136213 A1 (MERCK PATENT GMBH [DE]) 30 June 2022 (2022-06-30)<br>* page 1, line 10 - line 14 *<br>* page 2, line 14 - page 3, line 10 *<br>* page 3, line 24 - page 4, line 18 *<br>* page 5, line 26 - page 6, line 23 *<br>* page 8, line 9 - page 9, line 15 *<br>* page 12, line 12 - page 13, line 16 *<br>* page 16, line 23 - page 17, line 9 *<br>----- | 1-15 | |
| A | US 2022/017851 A1 (VANDIVER MICHAEL WAYNE [US] ET AL) 20 January 2022 (2022-01-20)<br>* paragraphs [0241], [0242], [0263], [0270], [0271] *<br>* paragraph [0409] - paragraph [0413] *<br>* paragraphs [0427], [0432] *<br>* figure 18 *<br>----- | 1-15 | |

TECHNICAL FIELDS
SEARCHED      (IPC)

The present search report has been drawn up for all claims

| Place of search | Date of completion of the search | Examiner |
|---|---|---|
| Berlin | 23 November 2023 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 8373

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2023

| Patent document cited in search report | | Publication date | Patent family member(s) | | Publication date |
|---|---|---|---|---|---|
| WO 2022136213 | A1 | 30-06-2022 | CN | 116635137 A | 22-08-2023 |
| | | | EP | 4263796 A1 | 25-10-2023 |
| | | | WO | 2022136213 A1 | 30-06-2022 |
| US 2022017851 | A1 | 20-01-2022 | AU | 2020223376 A1 | 22-07-2021 |
| | | | CA | 3124711 A1 | 20-08-2020 |
| | | | CN | 113439118 A | 24-09-2021 |
| | | | EP | 3924458 A1 | 22-12-2021 |
| | | | JP | 2022520793 A | 01-04-2022 |
| | | | KR | 20210127947 A | 25-10-2021 |
| | | | SG | 11202107441R A | 30-08-2021 |
| | | | US | 2022017851 A1 | 20-01-2022 |
| | | | US | 2022119757 A1 | 21-04-2022 |
| | | | US | 2022235311 A1 | 28-07-2022 |
| | | | US | 2022235312 A1 | 28-07-2022 |
| | | | WO | 2020168315 A1 | 20-08-2020 |
| | | | ZA | 202106720 B | 27-07-2022 |

EPO FORM P0459

For more details about this annex : see Official Journal of the European Patent Office, No. 12/82

**REFERENCES CITED IN THE DESCRIPTION**

**Patent documents cited in the description**

- WO 2007036291 A2

- WO 2009087087 A1